# EUROPEAN PATENT APPLICATION

(11) **EP 4 209 509 A1**
(43) Date of publication of application: **12.07.2023**
(21) Application number: 21863645.4
(22) Date of filing: 02.09.2021
(51) Int. Cl.: C07K 16/28, C12N 15/13, A61K 47/68, A61P 35/00

(54) **ROR1-TARGETING ANTIBODY OR ANTIGEN-BINDING FRAGMENT THEREOF, PREPARATION METHOD THEREFOR, AND APPLICATION THEREOF**

(30) Priority: 03.09.2020 CN 202010918328
(71) Applicant: Harbour Biomed (Shanghai) Co., Ltd, Shanghai 201203 (CN)
(72) Inventor: WANG, Zheng, Shanghai 201203 (CN); WANG, Yongqiang, Shanghai 201203 (CN); GAN, Xin, Shanghai 201203 (CN)
(74) Representative: Henderson, Helen Lee
(86) International application number: PCT/CN2021/116117
(87) International publication number: WO 2022/048581

(57) **Abstract**

The present invention discloses an ROR1-targeted antibody or an antigen-binding fragment thereof, a preparation method therefor and use thereof, wherein the ROR1-targeted antibody or the antigen-binding fragment thereof comprises a VL and a VH; the VL comprises the following CDRs or mutations thereof: VL CDR1, VL CDR2 and VL CDR3 as set forth in SEQ ID NOs: 69, 78 and 84, respectively; the VH comprises the following CDRs or mutations thereof: VH CDR1, VH CDR2 and VH CDR3 as set forth in SEQ ID NOs: 12, 33 and 53, respectively. The antibody or the antigen-binding fragment thereof described above has good binding affinity to ROR1, has almost no cross-reactivity with ROR2, has a KD value comparable to that of the reference antibody in the prior art, has a significant endocytosis effect, and has an ADCC effect in tumor cells.

## Description

The present application claims priority to Chinese Patent Application No. 2020109183287 filed on September 3, 2020, the content of which is incorporated herein by reference in its entirety.

### TECHNICAL FIELD

The present application relates to the field of biomedicine, and in particular to an ROR1-targeted antibody or an antigen-binding fragment thereof, a preparation method and use thereof.

### BACKGROUND

Recombinant receptor tyrosine kinase like orphan receptor 1 (ROR1), also known as NTRKR1, is an inactive transmembrane protein of tyrosine-protein kinase that is overexpressed in many tumors but hardly expressed in normal tissues. ROR1 comprises 937 amino acids in total, and the entire protein has a molecular weight of about 105 KDa (signal peptide (amino acids at positions 1-29) is excised). However, it has a short isotype that only comprises an extracellular domain consisting of 393 amino acids, and is localized in the cytoplasm or nucleus due to the lack of the transmembrane domain and signal peptide. In addition, a "truncated ROR1" has been reported in the literature, which only has a transmembrane domain on its membrane and an intracellular domain.

It is now well known that full-length ROR1, upon interacting with Wnt5a as a receptor, transduces the Wnt signaling pathway, thereby contributing to cell proliferation and migration in chronic lymphocytic leukemia (CLL) and epithelial-mesenchymal transition (EMT) in solid tumors.

Existing antibodies (such as the sequence R11H18L9 in the patent application WO_2016_094873_A2 of Emergent product development seattle, LLC.) have weak binding ability to ROR1, and have limited development space as monoclonal antibodies and bispecific antibodies in consideration of the limited expression of ROR1 antigen itself. Therefore, in the field, there is a lack of antibodies (especially fully human antibodies) that have high affinity for ROR1 antigen, do not cross-react with ROR2, and have better ADCC activity, endocytosis activity, etc.

### SUMMARY

The present invention addresses the technical problems that the ROR1-targeting antibodies in the field have weak binding ability to ROR1, have cross-reactivity with ROR2, have insufficient ADCC activity and endocytosis activity, and the like and provides an ROR1-targeted antibody or an antigen-binding fragment thereof, a preparation method and use thereof.

In order to solve the above technical problems, a first aspect of the present invention provides an ROR1-targeted antibody or an antigen-binding fragment thereof comprising a light chain variable region (VL) and/or a heavy chain variable region (VH); wherein,
the VL comprises the following CDRs or mutations thereof: VL CDR1 with an amino acid sequence as set forth in SEQ ID NO: 69, VL CDR2 with an amino acid sequence as set forth in SEQ ID NO: 78, and VL CDR3 with an amino acid sequence as set forth in SEQ ID NO: 84; and
the VH comprises the following CDRs or mutations thereof: VH CDR1 with an amino acid sequence as set forth in SEQ ID NO: 12, VH CDR2 with an amino acid sequence as set forth in SEQ ID NO: 33, and VH CDR3 with an amino acid sequence as set forth in SEQ ID NO: 53;
wherein the mutation is an insertion, deletion or substitution of 3, 2 or 1 amino acids on the amino acid sequences of the CDRs.

In the present application, "amino acid mutation" in the context like "insertion, deletion or substitution of 3, 2 or 1 amino acids" refers to mutations of amino acid sequenceof a variant as compared to the original amino acid sequence, including the insertion, deletion or substitution of amino acids on the basis of the original amino acid sequence. An exemplary explanation is that the mutations to the CDRs may comprise 3, 2 or 1 amino acid mutations, and that the same or different numbers of amino acid residues can be optionally selected for the mutations to those CDRs, e.g., 1 amino acid mutation to CDR1, and no amino acid mutation to CDR2 and CDR3. In the present application, the mutation may include mutations currently known to those skilled in the art, for example, mutations that may be made to an antibody during the production or application of the antibody, for example, mutations made to sites that may be present, especially those subjected to potential post-transcriptional modifications (PTMs) in CDR regions, including aggregation of antibodies, asparagine deamidation (NG, NS, NH, etc.) sensitive sites, aspartate isomerization (DG, DP) sensitive sites, N-glycosylation (N-{P}S/T) sensitive sites, oxidation sensitive sites, and the like.

Preferably, the VL CDR1 has mutations of 4, 3, 2 or 1 amino acid substitutions of T5S, I6M/V, N7S and N8S on the amino acid sequence as set forth in SEQ ID NO: 69, with an amino acid sequence preferably as set forth in any one of SEQ ID NOs: 70-74.

Preferably, the VL CDR2 has a mutation of an amino acid substitution of S4N on the amino acid sequence as set forth in SEQ ID NO: 78, with an amino acid sequence as set forth in SEQ ID NO: 79.

Preferably, the VLCDR3 has mutations of 2 or 1 amino acid substitutions of N4K/Q and S5L/I/A on the amino acid sequence as set forth in SEQ ID NO: 84, with an amino acid sequence preferably as set forth in any one of SEQ ID NOs: 85-87, SEQ ID NO: 184 and SEQ ID NO: 185.

Preferably, the VH CDR1 has mutations of 3, 2 or 1 amino acid substitutions of T3S, S6T/R/N and Y7F/S on the amino acid sequence as set forth in SEQ ID NO: 12, with an amino acid sequence preferably as set forth in any one of SEQ ID NOs: 13-18.

Preferably, the VH CDR2 has mutations of 4, 3, 2 or 1 amino acid substitutions of D3E, G4A, S5I/V/G and D6S/N on the amino acid sequence as set forth in SEQ ID NO: 33, with an amino acid sequence preferably as set forth in any one of SEQ ID NO: 32, SEQ ID NOs: 34-36 and SEQ ID NOs: 178-183.

Preferably, the VH CDR3 has mutations of 3, 2 or 1 amino substitutions of E1D, Q2L and L8I on the amino acid sequence as set forth in SEQ ID NO: 53, with an amino acid sequence preferably as set forth in SEQ ID NO: 52, 54 or 55.

The mutation T5S of VL CDR1 described above generally indicates that the amino acid T at position 5 on the amino acid sequence as set forth in SEQ ID NO: 69 is mutated to S. Other amino acid substitutions such as I6M/V, N7S and N8S, as well as mutations of VL CDR2, VL CDR3 and VH CDRs 1-3 and the like are deduced accordingly, and have meanings that should be understood by those skilled in the art. For example, the mutations N4K/Q and S5L/I/A of VL CDR3 can also be expressed as N92K/Q and S93L/I/A with mutation sites calculated cross the VL sequence, respectively, as shown in Table 10 below. Similarly, the mutations D3E and G4A of VH CDR2 can be expressed as D54E and G55A with mutation sites calculated cross the VH sequence, respectively.

Preferably, the VL CDR1 has an amino acid sequence as set forth in SEQ ID NO: 69, the VL CDR2 has an amino acid sequence as set forth in SEQ ID NO: 78, and the VL CDR3 has an amino acid sequence as set forth in SEQ ID NO: 84; or the VL CDR1 has an amino acid sequence as set forth in SEQ ID NO: 70, the VL CDR2 has an amino acid sequence as set forth in SEQ ID NO: 78, and the VL CDR3 has an amino acid sequence as set forth in SEQ ID NO: 85; or the VL CDR1 has an amino acid sequence as set forth in SEQ ID NO: 71, the VL CDR2 has an amino acid sequence as set forth in SEQ ID NO: 78, and the VL CDR3 has an amino acid sequence as set forth in SEQ ID NO: 84; the VL CDR1 has an amino acid sequence as set forth in SEQ ID NO: 72, the VL CDR2 has an amino acid sequence as set forth in SEQ ID NO: 78, and the VL CDR3 has an amino acid sequence as set forth in SEQ ID NO: 85; or the VL CDR1 has an amino acid sequence as set forth in SEQ ID NO: 69, the VL CDR2 has an amino acid sequence as set forth in SEQ ID NO: 78, and the VL CDR3 has an amino acid sequence as set forth in SEQ ID NO: 86; or the VL CDR1 has an amino acid sequence as set forth in SEQ ID NO: 73, the VL CDR2 has an amino acid sequence as set forth in SEQ ID NO: 79, and the VL CDR3 has an amino acid sequence as set forth in SEQ ID NO: 85; or the VL CDR1 has an amino acid sequence as set forth in SEQ ID NO: 74, the VL CDR2 has an amino acid sequence as set forth in SEQ ID NO: 78, and the VL CDR3 has an amino acid sequence as set forth in SEQ ID NO: 85; or the VL CDR1 has an amino acid sequence as set forth in SEQ ID NO: 69, the VL CDR2 has an amino acid sequence as set forth in SEQ ID NO: 78, and the VL CDR3 has an amino acid sequence as set forth in SEQ ID NO: 87; or the VL CDR1 has an amino acid sequence as set forth in SEQ ID NO: 73, the VL CDR2 has an amino acid sequence as set forth in SEQ ID NO: 78, and the VL CDR3 has an amino acid sequence as set forth in SEQ ID NO: 85; or the VL CDR1 has an amino acid sequence as set forth in SEQ ID NO: 72, the VL CDR2 has an amino acid sequence as set forth in SEQ ID NO: 78, and the VL CDR3 has an amino acid sequence as set forth in SEQ ID NO: 84; or the VL CDR1 has an amino acid sequence as set forth in SEQ ID NO: 73, the VL CDR2 has an amino acid sequence as set forth in SEQ ID NO: 78, and the VL CDR3 has an amino acid sequence as set forth in SEQ ID NO: 84; or the VL CDR1 has an amino acid sequence as set forth in SEQ ID NO: 70, the VL CDR2 has an amino acid sequence as set forth in SEQ ID NO: 78, and the VL CDR3 has an amino acid sequence as set forth in SEQ ID NO: 84; or the VL CDR1 has an amino acid sequence as set forth in SEQ ID NO: 69, the VL CDR2 has an amino acid sequence as set forth in SEQ ID NO: 78, and the VL CDR3 has an amino acid sequence as set forth in SEQ ID NO: 184; or the VL CDR1 has an amino acid sequence as set forth in SEQ ID NO: 69, the VL CDR2 has an amino acid sequence as set forth in SEQ ID NO: 78, and the VL CDR3 has an amino acid sequence as set forth in SEQ ID NO: 185; or the VL CDR1 has an amino acid sequence as set forth in SEQ ID NO: 72, the VL CDR2 has an amino acid sequence as set forth in SEQ ID NO: 78, and the VL CDR3 has an amino acid sequence as set forth in SEQ ID NO: 85.

Preferably, the VH CDR1 has an amino acid sequence as set forth in SEQ ID NO: 12, the VH CDR2 has an amino acid sequence as set forth in SEQ ID NO: 32, and the VH CDR3 has an amino acid sequence as set forth in SEQ ID NO: 52; or the VH CDR1 has an amino acid sequence as set forth in SEQ ID NO: 12, the VH CDR2 has an amino acid sequence as set forth in SEQ ID NO: 32, and the VH CDR3 has an amino acid sequence as set forth in SEQ ID NO: 53; or the VH CDR1 has an amino acid sequence as set forth in SEQ ID NO: 12, the VH CDR2 has an amino acid sequence as set forth in SEQ ID NO: 33, and the VH CDR3 has an amino acid sequence as set forth in SEQ ID NO: 53; or the VH CDR1 has an amino acid sequence as set forth in SEQ ID NO: 12, the VH CDR2 has an amino acid sequence as set forth in SEQ ID NO: 34, and the VH CDR3 has an amino acid sequence as set forth in SEQ ID NO: 53; or the VH CDR1 has an amino acid sequence as set forth in SEQ ID NO: 12, the VH CDR2 has an amino acid sequence as set forth in SEQ ID NO: 35, and the VH CDR3 has an amino acid sequence as set forth in SEQ ID NO: 53; or the VH CDR1 has an amino acid sequence as set forth in SEQ ID NO: 13, the VH CDR2 has an amino acid sequence as set forth in SEQ ID NO: 33, and the VH CDR3 has an amino acid sequence as set forth in SEQ ID NO: 53; or the VH CDR1 has an amino acid sequence as set forth in SEQ ID NO: 14, the VH CDR2 has an amino acid sequence as set forth in SEQ ID NO: 35, and the VH CDR3 has an amino acid sequence as set forth in SEQ ID NO: 53; or the VH CDR1 has an amino acid sequence as set forth in SEQ ID NO: 13, the VH CDR2 has an amino acid sequence as set forth in SEQ ID NO: 33, and the VH CDR3 has an amino acid sequence as set forth in SEQ ID NO: 54; or the VH CDR1 has an amino acid sequence as set forth in SEQ ID NO: 15, the VH CDR2 has an amino acid sequence as set forth in SEQ ID NO: 36, and the VH CDR3 has an amino acid sequence as set forth in SEQ ID NO: 53; or the VH CDR1 has an amino acid sequence as set forth in SEQ ID NO: 16, the VH CDR2 has an amino acid sequence as set forth in SEQ ID NO: 33, and the VH CDR3 has an amino acid sequence as set forth in SEQ ID NO: 53; or the VH CDR1 has an amino acid sequence as set forth in SEQ ID NO: 12, the VH CDR2 has an amino acid sequence as set forth in SEQ ID NO: 32, and the VH CDR3 has an amino acid sequence as set forth in SEQ ID NO: 55; or the VH CDR1 has an amino acid sequence as set forth in SEQ ID NO: 17, the VH CDR2 has an amino acid sequence as set forth in SEQ ID NO: 34, and the VH CDR3 has an amino acid sequence as set forth in SEQ ID NO: 53; or the VH CDR1 has an amino acid sequence as set forth in SEQ ID NO: 18, the VH CDR2 has an amino acid sequence as set forth in SEQ ID NO: 34, and the VH CDR3 has an amino acid sequence as set forth in SEQ ID NO: 53; or the VH CDR1 has an amino acid sequence as set forth in SEQ ID NO: 12, the VH CDR2 has an amino acid sequence as set forth in SEQ ID NO: 178, and the VH CDR3 has an amino acid sequence as set forth in SEQ ID NO: 53; or the VH CDR1 has an amino acid sequence as set forth in SEQ ID NO: 12, the VH CDR2 has an amino acid sequence as set forth in SEQ ID NO: 179, and the VH CDR3 has an amino acid sequence as set forth in SEQ ID NO: 53; or the VH CDR1 has an amino acid sequence as set forth in SEQ ID NO: 12, the VH CDR2 has an amino acid sequence as set forth in SEQ ID NO: 180, and the VH CDR3 has an amino acid sequence as set forth in SEQ ID NO: 53; or the VH CDR1 has an amino acid sequence as set forth in SEQ ID NO: 12, the VH CDR2 has an amino acid sequence as set forth in SEQ ID NO: 181, and the VH CDR3 has an amino acid sequence as set forth in SEQ ID NO: 53; or the VH CDR1 has an amino acid sequence as set forth in SEQ ID NO: 16, the VH CDR2 has an amino acid sequence as set forth in SEQ ID NO: 178, and the VH CDR3 has an amino acid sequence as set forth in SEQ ID NO: 53; or the VH CDR1 has an amino acid sequence as set forth in SEQ ID NO: 16, the VH CDR2 has an amino acid sequence as set forth in SEQ ID NO: 179, and the VH CDR3 has an amino acid sequence as set forth in SEQ ID NO: 53; or the VH CDR1 has an amino acid sequence as set forth in SEQ ID NO: 17, the VH CDR2 has an amino acid sequence as set forth in SEQ ID NO: 182, and the VH CDR3 has an amino acid sequence as set forth in SEQ ID NO: 53; or the VH CDR1 has an amino acid sequence as set forth in SEQ ID NO: 17, the VH CDR2 has an amino acid sequence as set forth in SEQ ID NO: 183, and the VH CDR3 has an amino acid sequence as set forth in SEQ ID NO: 53.

In a preferred embodiment, in the antibody or the antigen-binding fragment thereof, the VL comprises VL CDR1, VL CDR2, and VL CDR3 with amino acid sequences as set forth in the following table; and the VH comprises VH CDR1, VH CDR2 and VH CDR3 with amino acid sequences as set forth in the following table.

| **LCDR1 (SEQ ID NO:)** | **LCDR2 (SEQ ID NO:)** | **LCDR3 (SEQ ID NO:)** | **HCDR1 (SEQ ID NO:)** | **HCDR2 (SEQ ID NO:)** | **HCDR3 (SEQ ID NO:)** |
|---|---|---|---|---|---|
| 69 | 78 | 84 | 12 | 32 | 52 |
| 70 | 78 | 85 | 12 | 32 | 53 |
| 71 | 78 | 84 | 12 | 34 | 53 |
| 72 | 78 | 85 | 12 | 35 | 53 |
| 69 | 78 | 86 | 12 | 34 | 53 |
| 73 | 79 | 85 | 12 | 34 | 53 |
| 74 | 78 | 85 | 13 | 33 | 53 |
| 69 | 78 | 87 | 12 | 33 | 53 |
| 73 | 78 | 85 | 14 | 35 | 53 |
| 73 | 78 | 85 | 13 | 33 | 54 |
| 69 | 78 | 84 | 15 | 36 | 53 |
| 69 | 78 | 84 | 16 | 33 | 53 |
| 69 | 78 | 86 | 12 | 33 | 53 |
| 73 | 78 | 85 | 12 | 32 | 55 |
| 73 | 78 | 85 | 13 | 33 | 53 |
| 69 | 78 | 84 | 17 | 34 | 53 |
| 72 | 78 | 84 | 12 | 32 | 52 |
| 69 | 78 | 84 | 12 | 35 | 53 |
| 69 | 78 | 84 | 18 | 34 | 53 |
| 69 | 78 | 84 | 13 | 33 | 53 |
| 73 | 78 | 84 | 15 | 36 | 53 |
| 70 | 78 | 84 | 12 | 32 | 53 |
| 69 | 78 | 184 | 12 | 33 | 53 |
| 69 | 78 | 185 | 12 | 33 | 53 |
| 69 | 78 | 84 | 12 | 178 | 53 |
| 69 | 78 | 184 | 12 | 178 | 53 |
| 69 | 78 | 185 | 12 | 178 | 53 |
| 69 | 78 | 84 | 12 | 179 | 53 |
| 69 | 78 | 184 | 12 | 179 | 53 |
| 69 | 78 | 185 | 12 | 179 | 53 |
| 72 | 78 | 85 | 12 | 180 | 53 |
| 72 | 78 | 85 | 12 | 181 | 53 |
| 69 | 78 | 184 | 16 | 33 | 53 |
| 69 | 78 | 185 | 16 | 33 | 53 |
| 69 | 78 | 84 | 16 | 178 | 53 |
| 69 | 78 | 184 | 16 | 178 | 53 |
| 69 | 78 | 185 | 16 | 178 | 53 |
| 69 | 78 | 84 | 16 | 179 | 53 |
| 69 | 78 | 184 | 16 | 179 | 53 |
| 69 | 78 | 185 | 16 | 179 | 53 |
| 69 | 78 | 184 | 17 | 34 | 53 |
| 69 | 78 | 185 | 17 | 34 | 53 |
| 69 | 78 | 84 | 17 | 182 | 53 |
| 69 | 78 | 184 | 17 | 182 | 53 |
| 69 | 78 | 185 | 17 | 182 | 53 |
| 69 | 78 | 84 | 17 | 183 | 53 |
| 69 | 78 | 184 | 17 | 183 | 53 |
| 69 | 78 | 185 | 17 | 183 | 53 |

Preferably, the light chain variable region (VL) further comprises light chain variable region framework regions (VL FWRs), wherein the VL FWRs are preferably light chain variable region framework regions of a human antibody. More preferably, the VL FWRs include VL FWR1 with an amino acid sequence as set forth in SEQ ID NOs: 59-67 or mutations thereof, VL FWR2 with an amino acid sequence as set forth in SEQ ID NO: 76 or a mutation thereof, VL FWR3 with an amino acid sequence as set forth in SEQ ID NO: 81 or 82 or a mutation thereof, and VL FWR4 with an amino acid sequence as set forth in SEQ ID NOs: 89-91 or mutations thereof. Preferably, the heavy chain variable region (VH) further comprises heavy chain variable region framework regions (VH FWRs), wherein the VH FWRs are preferably heavy chain variable region framework regions of a human antibody, and more preferably, the VH FWRs include VH FWR1 with an amino acid sequence as set forth in SEQ ID NOs: 2-10 or mutations thereof, VH FWR2 with an amino acid sequence as set forth in SEQ ID NOs: 20-30 or mutations thereof, VH FWR3 with an amino acid sequence as set forth in SEQ ID NOs: 38-50 or mutations thereof, and VH FWR4 with an amino acid sequence as set forth in SEQ ID NO: 57 or a mutation thereof. The mutation is an insertion, deletion, substitution or repetition of 3, 2 or 1 amino acids on the amino acid sequences of the VH FWRs.

In a preferred embodiment, the VL comprises amino acid sequences as set forth in the following table or mutations thereof, and the VH comprises amino acid sequences as set forth in the following table or mutations thereof.

| VL (SEQ ID NO:) | VH (SEQ ID NO:) |
|---|---|
| 117 | 93 |
| 118 | 94 |
| 119 | 95 |
| 120 | 96 |
| 121 | 97 |
| 122 | 98 |
| 123 | 99 |
| 124 | 100 |
| 125 | 101 |
| 126 | 102 |
| 127 | 103 |
| 128 | 104 |
| 129 | 105 |
| 130 | 106 |
| 126 | 107 |
| 128 | 108 |
| 127 | 109 |
| 129 | 110 |
| 131 | 93 |
| 117 | 111 |
| 129 | 112 |
| 129 | 113 |
| 132 | 108 |
| 133 | 114 |
| 134 | 115 |
| 194 | 95 |
| 195 | 95 |
| 119 | 186 |
| 194 | 186 |
| 195 | 186 |
| 119 | 187 |
| 194 | 187 |
| 195 | 187 |
| 121 | 188 |
| 121 | 189 |
| 196 | 105 |
| 197 | 105 |
| 129 | 190 |
| 196 | 190 |
| 197 | 190 |
| 129 | 191 |
| 196 | 191 |
| 197 | 191 |
| 196 | 110 |
| 197 | 110 |
| 129 | 192 |
| 196 | 192 |
| 197 | 192 |
| 129 | 193 |
| 196 | 193 |
| 197 | 193 |

The mutation described above is a deletion, substitution or addition of one or more amino acid residues on the amino acid sequence of the VH and/or VL, and the amino acid sequence with the mutation has at least 85% sequence identity to the amino acid sequence of the VH and/or VL, and maintains or improves the binding of the antibody to ROR1, wherein the at least 85% sequence identity is preferably at least 90% sequence identity, more preferably at least 95%, 96%, 97% or 98% sequence identity, and most preferably at least 99% sequence identity.

In the present application, the amino acid sequences of the listed CDRs are all shown according to the Chothia scheme (the sequences in the claims of the present application are also shown according to the Chothia scheme). However, it is well known to those skilled in the art that the CDRs of an antibody can be defined in the art using a variety of methods, such as the Kabat scheme based on sequence variability (see Kabat et al., Sequences of Proteins of Immunological Interest, Fifth Edition, National Institutes of Health (U.S.), Bethesda, Maryland (1991)), and the Chothia scheme based on the location of the structural loop regions (see J Mol Biol 273: 927-948, 1997). In the present application, the Combined scheme comprising the Kabat scheme and the Chothia scheme can also be used to determine the amino acid residues in a variable domain sequence. The Combined scheme combines the Kabat scheme with the Chothia scheme to obtain a larger range, see Table 3 for details. It will be understood by those skilled in the art that unless otherwise specified, the terms "CDR" and "complementary determining region" of a given antibody or a region (e.g., variable region) thereof are construed as encompassing complementary determining regions as defined by any one of the above known schemes described herein. Although the scope claimed in the present invention is the sequences shown based on the Chothia scheme, the amino acid sequences corresponding to the other schemes for numbering CDRs shall also fall within the scope of the present invention.

Preferably, the ROR1-targeted antibody or the antigen-binding fragment thereof further comprises a heavy chain constant region and/or a light chain constant region.

More preferably, the heavy chain constant region is selected from hIgG1, hIgG2, hIgG3 and hIgG4 and mutations thereof, and the light chain constant region is selected from a κ chain and a λ chain and mutations thereof. More preferably, the heavy chain constant region is hIgG1 and the light chain constant region is a κ chain of a human antibody.

Still more preferably, the ROR1-targeted antibody or the antigen-binding fragment thereof is a full-length antibody; a Fab; a Fab'; an F(ab')₂; an Fv, preferably an scFv; a bispecific antibody (e.g., a bispecific antibody with a target such as OX40, 4-1BB and CD3); a multispecific antibody; a heavy-chain antibody or a single-domain antibody; or a monoclonal antibody or a polyclonal antibody derived from the above antibodies.

In the present application, a "Fab fragment" consists of one light chain and CH1 and the variable region of one heavy chain. The heavy chain of a Fab molecule cannot form disulfide bonds with another heavy chain molecule. An "Fc" region contains two heavy chain fragments comprising CH1 and CH2 domains of an antibody. The two heavy chain fragments are held together by two or more disulfide bonds and the hydrophobic interaction of the CH3 domains. The "Fab' fragment" contains one light chain and part of one heavy chain comprising the VH domain and the CH1 domain and the region between the CH1 and CH2 domains, so that interchain disulfide bonds can be formed between the two heavy chains of two Fab' fragments to provide an F(ab')₂ molecule. The "F(ab')₂ fragment" contains two light chains and two heavy chains comprising part of the constant region between the CH1 and CH2 domains, so that interchain disulfide bonds are formed between the two heavy chains. Thus, an F(ab')₂ fragment consists of two Fab' fragments held together by disulfide bonds between the two heavy chains. The term "Fv" refers to an antibody fragment consisting of the VL and VH domains of a single arm of an antibody, but lacks the constant region.

In the present application, the scFv (single-chain antibody fragment) may be a conventional single-chain antibody in the art, which comprises a heavy chain variable region, a light chain variable region, and a short peptide of 15-20 amino acids. In the scFv, the VL and VH domains are paired to form a monovalent molecule via a linker that enables them to produce a single polypeptide chain [see, e.g., Bird et al, Science 242:423-426 (1988) and Huston et al, Proc. Natl. Acad. Sci. USA 85:5879-5883 (1988)]. Such scFv molecules may have a general structure: NH2-VL-linker-VH-COOH or NH2-VH-linker-VL-COOH. An appropriate linker in the prior art consists of repeated G₄S amino acid sequences or a variant thereof. For example, linkers having the amino acid sequence (G₄S)₄ or (G₄S)₃ may be used, but a variant thereof may also be used. In the present application, the term "multispecific antibody" is used in its broadest sense to encompass antibodies having multi-epitope specificity. Those multispecific antibodies include, but are not limited to: an antibody comprising a heavy chain variable region (VH) and a light chain variable region (VL), the VH-VL unit having multi-epitope specificity; an antibody having two or more VL and VH regions, each of the VH-VL units binding to a different target or a different epitope on a same target; an antibody having two or more single variable regions, each of the single variable regions binding to a different target or a different epitope on a same target; full-length antibodies, antibody fragments, bispecific antibodies (diabodies), triabodies, antibody fragments linked together covalently or non-covalently, and the like.

In the present application, the monoclonal antibody or mAb or Ab refers to an antibody obtained from a single clonal cell strain, which is not limited to eukaryotic, prokaryotic, or phage clonal cell strains.

In the present application, the "heavy-chain antibody", also referred to as HCAb, refers to an antibody comprising only one heavy chain variable region (VHH) and two conventional CH2 and CH3 regions.

The "single-domain antibody", also referred to as "nanobody", refers to a VHH structure cloned from a heavy-chain antibody. It is the smallest unit known to be able to bind to a target antigen. Preferably, the ROR1-targeted antibody or the antigen-binding fragment thereof is a full-length antibody.

In a preferred embodiment, the full-length antibody comprises a heavy chain and a light chain, wherein the heavy chain comprises amino acid sequences as set forth in the following table or mutations thereof; and the light chain comprises amino acid sequences as set forth in the following table or mutations thereof.

| Light chain (SEQ ID NO:) | Heavy chain (SEQ ID NO:) |
|---|---|
| 160 | 136 |
| 161 | 137 |
| 162 | 138 |
| 163 | 139 |
| 164 | 140 |
| 165 | 141 |
| 166 | 142 |
| 167 | 143 |
| 168 | 144 |
| 169 | 145 |
| 170 | 146 |
| 171 | 147 |
| 172 | 148 |
| 173 | 149 |
| 169 | 150 |
| 171 | 151 |
| 170 | 152 |
| 172 | 153 |
| 174 | 136 |
| 160 | 154 |
| 172 | 155 |
| 172 | 156 |
| 175 | 151 |
| 176 | 157 |
| 177 | 158 |
| 206 | 138 |
| 207 | 138 |
| 162 | 198 |
| 206 | 198 |
| 207 | 198 |
| 162 | 199 |
| 206 | 199 |
| 207 | 199 |
| 164 | 200 |
| 164 | 201 |
| 208 | 148 |
| 209 | 148 |
| 172 | 202 |
| 208 | 202 |
| 209 | 202 |
| 172 | 203 |
| 208 | 203 |
| 209 | 203 |
| 208 | 153 |
| 209 | 153 |
| 172 | 204 |
| 208 | 204 |
| 209 | 204 |
| 172 | 205 |
| 208 | 205 |
| 209 | 205 |

The mutation described above is a deletion, substitution or addition of one or more amino acid residues on the amino acid sequence of the heavy and/or light chain, and the amino acid sequence with the mutation has at least 85% sequence identity to the amino acid sequence of the heavy and/or light chain, and maintains or improves the binding of the antibody to ROR1, wherein the at least 85% sequence identity is preferably at least 90% sequence identity, more preferably at least 95% sequence identity, and most preferably at least 99% sequence identity.

In order to solve the above technical problems, a second aspect of the present invention provides an isolated nucleic acid encoding the ROR1-targeted antibody or the antigen-binding fragment thereof according to the first aspect of the present invention.

The preparation method for the nucleic acid is a conventional preparation method in the art, and preferably comprises the following steps: obtaining a nucleic acid molecule encoding the above antibody by gene cloning technology, or obtaining a nucleic acid molecule encoding the above antibody by artificial complete sequence synthesis.

It is known to those skilled in the art that substitutions, deletions, alterations, insertions or additions may be appropriately introduced into the base sequence encoding the amino acid sequence of the above antibody to provide a polynucleotide homologue. The polynucleotide homologue of the present invention may be produced by substituting, deleting or adding one or more bases of a gene encoding the antibody sequence within a range in which the activity of the antibody is maintained.

In order to solve the above technical problems, a third aspect of the present invention provides a recombinant expression vector comprising the isolated nucleic acid according to the second aspect of the present invention.

The recombinant expression vector may be obtained by using conventional methods in the art, i.e., by linking the nucleic acid molecule of the present application to various expression vectors. The expression vector is any conventional vector in the art, provided that it can carry the aforementioned nucleic acid molecule.

Preferably, the expression vector comprises a eukaryotic cell expression vector and/or a prokaryotic cell expression vector.

In order to solve the above technical problems, a fourth aspect of the present invention provides a transformant comprising the recombinant expression vector according to the third aspect of the present invention.

The transformant may be prepared by using conventional methods in the art, e.g., by transforming the above recombinant expression vector into a host cell. The host cell of the transformant is any conventional host cell in the art, provided that it can enable the stable self-replication of the above recombinant expression vector and the effective expression of the nucleic acid carried in the vector. Preferably, the host cell is a prokaryotic cell and/or a eukaryotic cell, wherein the prokaryotic cell is preferably an *E. coli* cell such as TG1 and BL21 (expressing a single-chain antibody or Fab antibody), and the eukaryotic cell is preferably an HEK293 cell or a CHO cell (expressing a full-length IgG antibody). The preferred recombinant expression transformant of the present invention can be obtained by transforming the above recombinant expression plasmid into a host cell. The transformation method is a conventional transformation method in the art, preferably a chemical transformation method, a heat shock method or an electric transformation method.

In order to solve the above technical problems, a fifth aspect of the present invention provides a chimeric antigen receptor comprising the ROR1-targeted antibody or the antigen-binding fragment thereof according to the first aspect of the present invention.

In order to solve the above technical problems, a sixth aspect of the present invention provides a genetically modified cell comprising the chimeric antigen receptor according to the fifth aspect of the present invention.

Preferably, the genetically modified cell is a eukaryotic cell, preferably an isolated human cell, and more preferably an immune cell such as a T cell or an NK cell.

In order to solve the above technical problems, a seventh aspect of the present invention provides a method for preparing the ROR1-targeted antibody or the antigen-binding fragment thereof, which comprises the following steps: culturing the transformant according to the fourth aspect of the present invention, and obtaining the ROR1-targeted antibody or the antigen-binding fragment thereof from a culture.

In order to solve the above technical problems, an eighth aspect of the present invention provides an antibody-drug conjugate comprising a cytotoxic agent, and the ROR1-targeted antibody or the antigen-binding fragment thereof according to the first aspect of the present invention.

In order to solve the above technical problems, a ninth aspect of the present invention provides a pharmaceutical composition comprising the ROR1-targeted antibody or the antigen-binding fragment thereof according to the first aspect of the present invention, and/or the antibody-drug conjugate according to the eighth aspect of the present invention.

Preferably, the pharmaceutical composition further comprises an additional anti-tumor antibody as an active ingredient.

Preferably, the pharmaceutical composition further comprises a pharmaceutically acceptable carrier.

The pharmaceutically acceptable carrier may be a conventional carrier in the art, and the carrier may be any suitable physiologically or pharmaceutically acceptable auxiliary material. The pharmaceutically acceptable auxiliary material is one conventional in the art, and preferably comprises a pharmaceutically acceptable excipient, a filler, a diluent, or the like. More preferably, the pharmaceutical composition comprises 0.01%-99.99% of the above protein and/or the above antibody-drug conjugate and 0.01%-99.99% of a pharmaceutically acceptable carrier, the percentage is the mass percentage of the pharmaceutical composition.

The route of administration for the pharmaceutical composition described herein is preferably parenteral administration, injection administration or oral administration. The injection administration preferably includes intravenous injection, intramuscular injection, intraperitoneal injection, intradermal injection, subcutaneous injection or the like. The pharmaceutical composition is in any conventional dosage form in the art, preferably in the form of a solid, semisolid or liquid, i.e., it may be an aqueous solution, a non-aqueous solution or a suspension, more preferably a tablet, capsule, granule, injection, infusion, or the like. More preferably, it is administered intravascularly, subcutaneously, intraperitoneally or intramuscularly. Preferably, the pharmaceutical composition may also be administered as an aerosol or a coarse spray, i.e., administered nasally; or administered intrathecally, intramedullarily or intraventricularly. More preferably, the pharmaceutical composition may also be administered transdermally, percutaneously, topically, enterally, intravaginally, sublingually or rectally. The pharmaceutical composition of the present invention may be formulated into various dosage forms as required, and can be administered by a physician in the light of the patient's type, age, weight, and general disease state, route of administration, etc. The administration may be performed, for example, by injection or other therapeutic modalities.

The dose level at which the pharmaceutical composition of the present invention is administered can be adjusted depending on the amount of the composition to achieve the desired diagnostic or therapeutic outcome. The administration regimen may also be a single injection or multiple injections, or an adjusted one. The selected dose level and regimen is appropriately adjusted depending on a variety of factors including the activity and stability (i.e., half-life) of the pharmaceutical composition, the formulation, the route of administration, combination with other drugs or treatments, the disease or disorder to be detected and/or treated, and the health condition and previous medical history of the subject to be treated.

A therapeutically effective dose for the pharmaceutical composition of the present invention may be estimated initially in cell culture experiments or animal models such as rodents, rabbits, dogs, pigs and/or primates. Animal models can also be used to determine the appropriate concentration range and route of administration, and subsequently an effective dose and a route of administration in humans. In general, the determination of and adjustment to the effective amount or dose to be administered and the assessment of when and how to make such adjustments are known to those skilled in the art.

For combination therapy, the ROR1-targeted antibody or the antigen-binding fragment thereof described above, the antibody-drug conjugate described above and/or an additional therapeutic or diagnostic agent may each be used as a single agent for use within any time frame suitable for performing the intended treatment or diagnosis. Thus, these single agents may be administered substantially simultaneously (i.e., as a single formulation or within minutes or hours) or sequentially.

For additional guidance regarding formulations, doses, dosage regimens, and measurable therapeutic outcomes, see Berkow et al. (2000) The Merck Manual of Medical Information and Merck & Co. Inc., Whitehouse Station, New Jersey; Ebadi (1998) CRC Desk Reference of Clinical Pharmacology, etc.

In order to solve the above technical problems, a tenth aspect of the present invention provides use of the ROR1-targeted antibody or the antigen-binding fragment thereof according to the first aspect of the present invention, the chimeric antigen receptor according to the fifth aspect of the present invention, the genetically modified cell according to the sixth aspect of the present invention, the antibody-drug conjugate according to the eighth aspect of the present invention, and/or the pharmaceutical composition according to the ninth aspect of the present invention in the preparation of a medicament for the prevention and/or treatment of a cancer.

Preferably, the cancer is colon cancer, lung cancer, prostate cancer, liver cancer, kidney cancer, pancreatic cancer, breast cancer, cervical cancer, ovarian cancer, thyroid tumor, chronic lymphocytic leukemia, acute lymphocytic leukemia, acute myeloid leukemia, non-Hodgkin's lymphoma, chronic myeloid lymphocytic leukemia, multiple myeloma, melanoma, colorectal cancer, glioblastoma and/or endometrial cancer.

In order to solve the above technical problems, an eleventh aspect of the present invention provides a kit comprising the ROR1-targeted antibody or the antigen-binding fragment thereof according to the first aspect of the present invention, the chimeric antigen receptor according to the fifth aspect of the present invention, the genetically modified cell according to the sixth aspect of the present invention, the antibody-drug conjugate according to the eighth aspect of the present invention, and/or the pharmaceutical composition according to the ninth aspect of the present invention.

Preferably, the kit further comprises (i) a device for administering the antibody or the antigen-binding fragment thereof, the antibody-drug conjugate or the pharmaceutical composition; and/or (ii) instructions.

In order to solve the above technical problems, the present invention further provides a method for detecting ROR1 in a sample, which comprises conducting a detection using the ROR1-targeted antibody or the antigen-binding fragment thereof according to the first aspect of the present invention.

Preferably, the detection is a diagnosis.

In order to solve the above technical problems, the present invention further provides use of the ROR1-targeted antibody or the antigen-binding fragment thereof according to the first aspect of the present invention, the chimeric antigen receptor according to the fifth aspect of the present invention, the genetically modified cell according to the sixth aspect of the present invention, the antibody-drug conjugate according to the eighth aspect of the present invention, and/or the pharmaceutical composition according to the ninth aspect of the present invention in the diagnosis, prevention and/or treatment of a cancer. Preferably, the cancer is as described in the tenth aspect of the present invention.

In order to solve the above technical problems, the present invention further provides a kit of parts comprising a kit A and a kit B, wherein the kit A comprises the ROR1-targeted antibody or the antigen-binding fragment thereof according to the first aspect of the present invention, the chimeric antigen receptor according to the fifth aspect of the present invention, the genetically modified cell according to the sixth aspect of the present invention, the antibody-drug conjugate according to the eighth aspect of the present invention, and/or the pharmaceutical composition according to the ninth aspect of the present invention; and the kit B is an additional drug, preferably a macromolecular drug such as an anti-cancer (anti-tumor) antibody or a pharmaceutical composition comprising the additional anti-cancer (anti-tumor) antibody, or a micromolecular drug.

The kit A and the kit B may be used simultaneously, or the kit A may be used prior to the use of the kit B, or the kit B may be used prior to the use of the kit A. The sequence of use can be determined according to actual requirements in a specific application.

In order to solve the above technical problems, the present invention further provides a method for treating a cancer in a subject in need, comprising administering to the subject in need the ROR1-targeted antibody or the antigen-binding fragment thereof according to the first aspect of the present invention, the chimeric antigen receptor according to the fifth aspect of the present invention, the genetically modified cell according to the sixth aspect of the present invention, the antibody-drug conjugate according to the eighth aspect of the present invention, and/or the pharmaceutical composition according to the ninth aspect of the present invention. Preferably, the cancer is as described in the tenth aspect of the present invention.

Preferably, the method further comprises administering an additional drug, preferably a macromolecular drug such as an additional anti-cancer (anti-tumor) antibody or a pharmaceutical composition comprising the additional anti-cancer (anti-tumor) antibody, or a micromolecular drug, for example, in combination with an additional drug, e.g., in combination with a chemotherapeutic agent or the like.

In the present application, unless otherwise defined, the scientific and technical terms used herein have the meanings generally understood by those skilled in the art. In addition, the laboratory operations of cell culture, molecular genetics, nucleic acid chemistry and immunology used herein are the conventional procedures widely used in the corresponding fields. Meanwhile, in order to better understand the present invention, the definitions and explanations of the relevant terms are provided below.

In the present application, the term "variable" generally refers to the fact that certain portions of the sequences of the variable domains of antibodies vary considerably, resulting in the binding and specificity of various particular antibodies to their particular antigens. However, variability is not evenly distributed throughout the variable region of the antibody. It is concentrated in three segments in each of the light chain and heavy chain variable regions called complementary determining regions (CDRs) or hypervariable regions (HVRs). The more highly conserved portions of the variable domains are called frameworks (FWRs). The variable domains of native heavy and light chains each comprise four FWRs largely in a β-sheet configuration. The FRs are connected by three CDRs to form a loop connection, and in some cases to form part of a β-sheet structure. The CDRs in each chain are held in close proximity by the FWRs and form, together with the CDRs from the other chain, antigen-binding sites of the antibody. The constant regions are not directly involved in the binding of the antibody to antigens, but they exhibit different effector functions, for example, being involved in antibody-dependent cellular cytotoxicity of the antibody.

The three-letter codes and single-letter codes for amino acids used in the present application are known to those skilled in the art, or are described in J. Biol. Chem, 243, p3558 (1968).

As used herein, the term "include/includes/including" or "comprise/comprises/comprising" is intended to mean that a composition and a method include the elements described but does not exclude other elements; but the case of "consist/consists/consisting of" is also included as the context dictates.

The term "antibody" described herein may include an immunoglobulin, which is of a tetrapeptide chain structure formed by connection between two identical heavy chains and two identical light chains by interchain disulfide bonds. Immunoglobulins differ in amino acid composition and arrangement of their heavy chain constant regions and therefore in their antigenicity. Accordingly, immunoglobulins can be classified into five classes, or isotypes of immunoglobulins, namely IgM, IgD, IgG, IgA and IgE, with their corresponding heavy chains being the µ, δ, γ, α and ε chains, respectively. The Ig of the same class can be divided into different subclasses according to the differences in amino acid composition of the hinge regions and the number and location of disulfide bonds in the heavy chains; for example, IgG can be divided into IgG1, IgG2, IgG3, and IgG4. Light chains are classified into κ or λ chains by the difference in the constant regions. Each of the five classes of Ig can have a κ chain or a λ chain. In the present application, the light chain variable region of the antibody of the present application may further comprise a light chain constant region comprising a human κ or λ chain or a variant thereof. In the present application, the heavy chain variable region of the antibody of the present application may further comprise a heavy chain constant region comprising human IgG1, IgG2, IgG3 or IgG4 or a variant thereof.

In light chains and heavy chains, the variable region and constant region are linked by a "J" region of about 12 or more amino acids, and the heavy chain further comprises a "D" region of about 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain CL. The constant region of the antibody can mediate the binding of immunoglobulins to host tissues or factors, including the binding of various cells of the immune system (e.g., effector cells) to the first component (C1q) of classical complement system. The sequences of about 110 amino acids of the heavy and light chains of the antibody near the N-terminus vary considerably and thus are referred to as variable regions (V regions); the remaining amino acid sequences near the C-terminus are relatively stable and thus are referred to as constant regions (C regions). The variable regions comprise 3 hypervariable regions (HVRs) and 4 framework regions (FWRs) with relatively conservative sequences. The 3 hypervariable regions determine the specificity of the antibody and thus are also referred to as complementary determining regions (CDRs). Each of the light chain variable regions (VLs) and the heavy chain variable region (VHs) consists of 3 CDR regions and 4 FWR regions arranged from the amino-terminus to the carboxyl-terminus in the following order: FWR1, CDR1, FWR2, CDR2, FWR3, CDR3, and FWR4. The 3 CDR regions of the light chain refer to VL CDR1, VL CDR2 and VL CDR3; and the 3 CDR regions of the heavy chain refer to VH CDR1, VH CDR2 and VH CDR3.

The term "human antibody" includes antibodies having variable and constant regions of human germline immunoglobulin sequences. The human antibody of the present application may include amino acid residues not encoded by human germline immunoglobulin sequences (e.g., mutations introduced by *in vitro* random or site-directed mutagenesis or *in vivo* somatic mutation). However, the term "human antibody" does not include antibodies in which CDR sequences derived from the germline of another mammalian species, such as a mouse, have been grafted into human framework sequences (i.e., "humanized antibodies").

As used herein, the term "specificity" with respect to an antibody means that an antibody recognizes a specific antigen but does not substantially recognize or bind to other molecules in a sample. For example, an antibody specifically binding to an antigen from one species may also bind to the antigen from one or more species. However, such interspecific cross-reactivity per se does not change the classification of antibodies by specificity. In another example, an antibody specifically binding to an antigen may also bind to the antigen in different allelic forms. However, such cross-reactivity per se does not change the classification of antibodies by specificity. In some cases, the term "specificity" or "specific binding" may be used to refer to the interaction of an antibody, a protein or a peptide with a second chemical substance, meaning that the interaction is dependent on the presence of a particular structure (e.g., an antigenic determinant or epitope) in the chemical substance; for example, an antibody generally recognizes and binds to a particular protein structure rather than a protein. If an antibody has specificity to an epitope "A", then in a reaction containing labeled "A" and the antibody, the presence of a molecule containing the epitope A (or free, unlabeled A) will reduce the amount of labeled A bound to the antibody.

In the present application, the term "antigen-binding fragment" refers to an antigen-binding fragment and an antibody analog of the antibody, which generally include at least a portion of the antigen-binding region or variable region (e.g., one or more CDRs) of a parental antibody. The antibody fragment retains at least some of the binding specificities of the parental antibody. Generally, the antibody fragment retains at least 10% of the binding activity of the parental antibody when the activity is expressed on a molar basis. Preferably, the antibody fragment retains at least 50%, 60%, 70%, 80%, 90%, 95%, 99%, or 100% or more of the binding affinity of the parental antibody for the target. Examples of the antigen-binding fragment include, but are not limited to: Fab, Fab', F(ab')₂, Fv fragments, linear antibodies, single (heavy) chain antibodies, nanobodies, domain antibodies, multispecific antibodies and the like. Engineered antibody variants are reviewed in Holliger and Hudson (2005) Nat. Biotechnol. 23: 1126-1136. The term "chimeric antigen receptor" or "CAR" used herein includes extracellular domains (extracellular binding domains), hinge domains, transmembrane domains (transmembrane regions) capable of binding to antigens and polypeptides that pass a cytoplasmic signal to a domain (i.e., an intracellular signal domain). The hinge domain may be considered as a part for providing flexibility to an extracellular antigen-binding region. The intracellular signal domain refers to a protein that transmits information into a cell via a determined signaling pathway by generating a second messenger to regulate the activity of the cell, or a protein that functions as an effector by corresponding to such a messenger. It generates a signal that can promote the immune effector function of a cell of the CAR (e.g., a CAR-T cell). The intracellular signal domain includes a signaling domain, and may also include a co-stimulatory intracellular domain derived from a co-stimulatory molecule.

"Identity" or "mutation" refers to sequence similarity between two polynucleotide sequences or between two polypeptide sequences. When positions in two compared sequences are all occupied by the same base or amino acid monomer subunit, for example, if a position in each of two DNA molecules is occupied by adenine, the molecules are homologous at that position. The identity percentage between two sequences is a function of the number of matched or homologous positions shared by the two sequences divided by the number of the compared positions × 100%. For example, when sequences are optimally aligned, if 6 out of 10 positions in two sequences match or are homologous, the two sequences are 60% homologous. In general, the comparison is made when two aligned sequences give the greatest identity percentage.

The terms "polypeptide", "peptide" and "protein" (if single-stranded) are used interchangeably in the present application. The terms "nucleic acid", "nucleic acid sequence", "nucleotide sequence" or "polynucleotide sequence", and "polynucleotide" are used interchangeably.

The term "vector" used herein is a composition that comprises an isolated nucleic acid and is useful for delivering the isolated nucleic acid to the interior of a cell. Many vectors are known in the art, including but not limited to, linear polynucleotides, polynucleotides associated with ionic or amphiphilic compounds, plasmids, and viruses. Thus, the term "vector" includes autonomously replicating plasmids or viruses. The term should also be construed as including non-plasmid and non-viral compounds that facilitate the transfer of nucleic acids into cells, such as polylysine compounds and liposomes. Examples of viral vectors include, but are not limited to, adenoviral vectors, adeno-associated viral vectors, retroviral vectors, etc.

The expressions "cell" and "cell line" used in the present application are used interchangeably and all such designations include progeny. The term "host cell" refers to a cell to which a vector can be introduced, including, but not limited to, prokaryotic cells such as *E. coli*, fungal cells such as yeast cells, or animal cells such as fibroblasts, CHO cells, COS cells, NSO cells, HeLa cells, BHK cells, HEK 293 cells, or human cells.

The term "transfection" refers to the introduction of an exogenous nucleic acid into a eukaryotic cell. Transfection may be accomplished by a variety of means known in the art, including calcium phosphate-DNA co-precipitation, DEAE-dextran mediated transfection, polybrene-mediated transfection, electroporation, microinjection, liposome fusion, lipofection, protoplast fusion, retroviral infection, and biolistics.

The term "immune cell" refers to a cell that can elicit an immune response. The "immune cell" and other grammatical variations thereof may refer to an immune cell of any origin. The "immune cell" includes, for example, white blood cells (leukocytes) and lymphocytes (T cells, B cells, and natural killer (NK) cells) derived from hematopoietic stem cells (HSCs) produced in the bone marrow, and bone marrow-derived cells (neutrophils, eosinophils, basophils, monocytes, macrophages, dendritic cells). The term "immune cell" may also refer to a human or non-human immune cell. For example, the immune cells may be derived from the blood, such as autologous T cells, allogeneic T cells, autologous NK cells and allogeneic NK cells, or from cell lines, such as NK cell lines prepared by infection with the EBV virus, NK cells obtained by induced differentiation of embryonic stem cells and iPSCs, as well as NK92 cell lines.

As used herein, the term "T cell" refers to a class of lymphocytes that are matured in the thymus. T cells play an important role in cell-mediated immunity and are different from other lymphocytes (e.g., B cells) in that T cell receptors are present on the cell surface. "T cell" includes all types of immune cells expressing CD3, including T helper cells (CD4⁺ cells), cytotoxic T cells (CD8⁺ cells), natural killer T cells, T regulatory cells (Tregs), and γ-δT cells. "Cytotoxic cells" include CD8⁺ T cells, natural killer (NK) cells and neutrophils, which are capable of mediating a cytotoxic response. As used herein, the term "NK cell" refers to a class of lymphocytes that originate in the bone marrow and play an important role in the innate immune system. NK cells provide a rapid immune response against virus-infected cells, tumor cells or other stressed cells, even in the absence of antibodies and major histocompatibility complexes on the cell surface.

The term "optional", "optionally", "any" or "any one of" means that the event or circumstance subsequently described may, but not necessarily, occur, and that the description includes instances where the event or circumstance occurs or does not occur. For example, "optionally comprising 1 antibody heavy chain variable region" means that the antibody heavy chain variable region of a particular sequence may, but not necessarily, be present. As used herein, "a" and "an" are used in the present invention to refer to one or more grammatical objects. Unless otherwise specifically stated in the content, the term "or" is used in the present invention to refer to, and is used interchangeably with the term "and/or". The "about" and "approximately" shall generally mean an acceptable degree of error in the measured quantity in view of the nature or accuracy of the measurement. Exemplary degrees of error are typically within 10% thereof and more typically within 5% thereof. The method and composition disclosed in the present invention encompass polypeptides and nucleic acids having a specified sequence, a variant sequence, or a sequence substantially identical or similar thereto, e.g., a sequence that is at least 85%, 90%, 95%, 99% or more identical to the sequence specified. In the context of amino acid sequences, the term "substantially identical" is used herein to refer to a first amino acid sequence.

As used herein, the term "EC₅₀" refers to the concentration for 50% of maximal effect, i.e., the concentration that can cause 50% of the maximal effect.

As used herein, the terms "cancer" and "cancer patient" are intended to include all types of cancerous growths or oncogenic processes, metastatic tissues or malignantly transformed cells, tissues or organs, regardless of their histopathological types or stages of invasiveness. Examples include, but are not limited to, solid tumors, hematologic cancer, soft tissue tumors and metastatic lesions.

It will be understood by those skilled in the art that products comprising the ROR1-targeted antibody or the antigen-binding fragment thereof described herein, e.g., CAR-T, TCR-T, bispecific antibodies, multispecific antibodies, ADC and the like, shall fall within the scope of the present invention.

The above preferred conditions may be combined arbitrarily to obtain preferred embodiments of the present invention on the basis of the general knowledge in the art.

The reagents and starting materials used in the present invention are commercially available.

The positive and progressive effects of the present invention are as follows: the ROR1-targeted antibody or the antigen-binding fragment thereof described herein has good binding affinity for hROR1 and cynoROR1 and has almost no cross-reactivity with ROR2. The ROR1-targeted antibody or the antigen-binding fragment thereof described herein has a KD value comparable to that of the reference antibody in the prior art, and shows significant endocytosis effect. The ROR1-targeted antibody or the antigen-binding fragment thereof described herein has an ADCC effect in tumor cells, and has no significant CDC/ADCP effects. Based on its application in the direction of ADCC, monoclonal antibodies may be developed in the future. In addition, the removal mutations at most of the PTM sites in the ROR1-targeted antibody or the antigen-binding fragment thereof described herein do not affect the binding ability of the antibody, which is more conducive to the downstream production of patent medicines. In a preferred embodiment, the ROR1-targeted antibody or the antigen-binding fragment thereof described herein, when bound to a tumor cell line, has an EC₅₀ value almost 1 log higher than that of the existing antibody in the prior art.

### BRIEF DESCRIPTION OF THE DRAWINGS

FIG. 1 illustrates a workflow for constructing a phage library.
FIG. 2 illustrates the results of ELISA screening in Example 3.
FIG. 3 illustrates the results of FACS screening in Example 3.
FIG. 4 illustrates the ELISA assays on the binding of recombinant antibodies to a 293T-hROR1 cell line; wherein, A shows the results for PR001352-PR001371 and B shows the results for PR001415-PR001419.
FIG. 5 illustrates the ELISA assays on the binding of recombinant antibodies to a CHO-K1-cynoROR1 cell line; wherein, A shows the binding data of PR001352-PR001371 and B shows the binding data of PR001415-PR001419.
FIG. 6 illustrates the ELISA assays on the binding of recombinant antibodies to an A549 cell line; wherein, A shows the binding data of PR001352-PR001371 and B shows the binding data of PR001415-PR001419.
FIG. 7 illustrates the data from endocytosis of PR001352-PR001371 and PR001415-PR001419 on HEK293T-hROR1 cells.
FIG. 8 illustrates the data from endocytosis of PR001353, PR001354 and PR001357 at lower concentrations on HEK293T-hROR1 cells.
FIG. 9 illustrates the ADCC effects of recombinant antibodies on an A549 cell line; wherein, A and B shows the results for PR001353, PR001354, PR001355, PR001357, PR001359 and PR001362 of donors 1 and 2, respectively.
FIG. 10 illustrates the CDC effects of recombinant antibodies on HEK293T-hROR1.
FIG. 11 illustrates the ADCP effects of recombinant antibodies on A549 cells.

### DETAILED DESCRIPTION

The present invention is further illustrated by the following examples, which are not intended to limit the present invention. Experimental procedures without specified conditions in the following examples are performed in accordance with conventional procedures and conditions, or in accordance with instructions.

### Example 1. Preparation of Antigens and Stably Transfected Cell Strains

a. Preparation of antigens and other proteins
   Human ROR1-ECD-Fc (abbreviated as hROR1-ECD-Fc) protein was purchased from Acrobiosystems, Cat#: RO1-H5250;
   Human ROR1-ECD-his (abbreviated as hROR1-ECD-his) protein was purchased from Acrobiosystems, Cat#: RO1-H522y;
   Biotinylated ROR1 (Biotin-ROR1) was purchased from Acrobiosystems, Cat#: RO1-H821y; hROR2ECD-his was purchased from Acrobiosystems, Cat#: RO2-H52E5; and mROR1ECD-his was purchased from Acrobiosystems, Cat#: RO1-M5221.
b. Preparation of stably transfected cell strains
   HEK293T-hROR1 (human ROR1) was purchased from KYinno, Cat#: KC-1018;
   CHO-K1-cynoROR1 (cynomolgus ROR1) stably transfected cell strains were prepared in the laboratory by the following procedures:
      CHO-K1 cell suspension with a density of 50000 cells/mL was seeded in a 6-well plate at 2 mL/well and incubated overnight, followed by the addition of polybrene (Shanghai GeneChem, Cat#: REVG0001, 10 mg/mL) to give a final concentration of 4 µg/mL. The mixture was well mixed, and 10 µL of virus (LV-cynoROR1, purchased from Shanghai GeneChem, Cat#: 42582-1, titer: 1e9/mL, 50 µL/vial) was added. The mixture was well mixed and then incubated at 37 °C for 8 h. Thereafter, the supernatant was discarded, the medium was replaced by a fresh medium, and then the cells were cultured at 37 °C. After 24 h, the cells were resuspended and digested. Then the cells were diluted to 5 cells/mL with a complete medium containing 8 µg/mL puromycin, seeded in 10 96-well plates at 100 µL/well, and cultured at 37 °C for 10 days. Single clones were picked for FACS verification. After the verification, the verified clones were further expanded, and then cryopreserved.

### Example 2. Phage Display

### a. Construction of phage library for H2L2 mice immunized with ROR1

Immunization of mice: The H2L2 mice were immunized with hROR1-ECD-Fc fusion protein (purchased from Harbour BioMed, granted patent EP2379727B1). Specifically, each mouse was injected with 50 µg of the above fusion protein in combination with CFA for the first time, and then subjected to 7 booster immunizations with Ribi adjuvant on days 15, 29, 43, 57, 71, 86 and 100. Blood was collected on days 50, 78 and 107 for testing. The last booster immunization was performed 3 days prior to fusion, i.e., on day 137.

Spleen cells were collected from the above immunized mice, and splenic B cells were isolated therefrom. RNA was extracted using TRIZOL according to the product instructions (Thermofisher, Cat. No. 15596018), and RT-PCR was conducted according to the product instructions (Thermofisher, Cat. No. 11756500). The VH (heavy chain variable region) and VL (light chain variable region) were obtained by amplification by first PCR using cDNA as a template, and scFv fragments were obtained by overlap PCR.

The reaction system and reaction program for the first PCR described above are shown in Table 1-1 and Table 1-2 below:

**Table 1-1. Reaction system**

| Total RNA-cDNA | 1 µL/1 µL water as a negative control |
|---|---|
| 5× Q5 reaction buffer | 10 µl |
| 2.5mM dNTPs | 4µl |
| Forward primer (10 µM) | 2µl |
| Reverse primer (10 µM) | 2µl |
| Q5 DNA polymerase | 0.5µl |
| ddH₂O | 30.5µl |
| Total volume | 50 µl |

**Table 1-2. PCR reaction program**

| | | |
|---|---|---|
| 98°C | 30s | |
| 98°C | 10s | 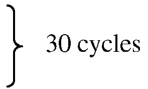 |
| 64°C | 30s | |
| 72°C | 30s | |
| 72°C | 10mins | |

The reaction system and the reaction program for the overlap PCR are shown in Table 2-1 and Table 2-2 below:

**Table 2-1. Reaction system**

| | |
|---|---|
| VH mixture (50 ng) | X µl |
| Vκ mixture (50 ng) | Y µl |
| 5× Q5 reaction buffer | 5µl |
| 5× Q5 GC enhancer | 5µl |
| 2.5mM dNTPs | 2µl |
| Q5 DNA polymerase | 0.25µl |
| ddH₂O Up to | 25µl |

In the above table, since the concentrations of the samples were different, the volume was converted by mass. Therefore, the volumes of VH and VL in each reaction were different, which were represented here by X and Y, respectively. The mass of each gene in the reaction system was ensured to be 50 ng, and the total volume was finally supplemented to 25 µL with water.

**Table 2-2. PCR reaction program**

| | | |
|---|---|---|
| 98°C | 30s | |
| 98°C | 10s | 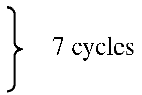 |
| 68°C | 30s | |
| 72°C | 30s | |
| 72°C | 5mins | |

After 7 cycles of reaction, the primers *SfiF* (1 µL)/*Sfi*R (1 µL) were added to perform overlap PCR, and the reaction program is shown in Table 2-3 below.

**Table 2-3. PCR reaction program**

| | | |
|---|---|---|
| 98°C | 30s | |
| 98°C | 10s | 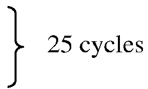 |
| 66°C | 30s | |
| 72°C | 40s | |
| 72°C | 5mins | |

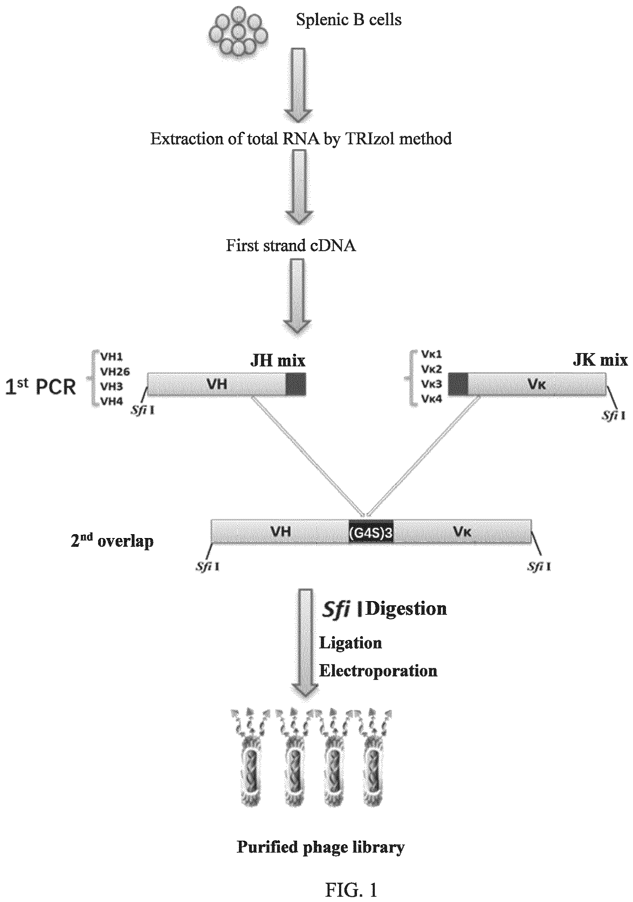

The ligation product obtained above was transformed into MC1061F' (Lucigen, Cat#: 60512-1), and then a phage library was prepared (the workflow and result of the construction of the phage library are shown in FIG. 1). Three rounds of bio-panning (the steps are shown in b below) were performed, and screening was performed by ELISA (the steps are shown in c below) with biotinylated ROR1. All the selected candidates (hits) were sent for sequencing (the steps are described below), and specific clones were produced.

### b. Bio-panning

The phage library (1E13 phage particles) obtained above was depleted with streptavidin (SA)-coated beads (SA beads, Thermofisher, Cat#: 11206), and then incubated with SA-Bio-ROR1 beads at room temperature (refer to product instructions, Thermofisher, Cat#: 11206), followed by washing 14 times with 1× PBST. After washing, a citrate buffer (pH 3.0) was added to the magnetic bead/phage mixture, and the mixture was incubated at room temperature for 10 min. Then, a Tris-HCl neutralization buffer (pH 9.0) was added, and the supernatant was then taken to infect 10 mL of MC1061F' cells at 37 °C for 15 min. The infected cells were subjected to limiting dilution and then plated on a plate with 2× YT (containing 100 µg/mL Amp). The plate was incubated overnight at 37 °C, and colonies were formed. Thereafter, 50 µL of M13K07 helper phages (NEB, Cat#: N0315S) at 2E12/mL were added to the infected cells described above, and the incubation was continued at 37 °C for 15 min. Finally, 100 mL of 2× YT (containing 100 µg/mL Amp, 50 µg/mL Kan and 1 mM IPTG) was added, and the mixture was incubated overnight at 30 °C. 100 mL of the overnight culture was centrifuged and filtered through a 0.22 µM filter, and 7 mL of 20% PEG6000 and 2.5 M NaCl were added to 35 mL of the supernatant (in a volume ratio of 1:5), followed by centrifugation at 8000 rpm for 30 min at 4 °C. The phage pellet obtained after centrifugation was resuspended in 1 mL of 1× PBST and centrifuged at 10,000 rpm for 5 min to discard debris. The resulting supernatant (phage particles) was transferred to a new tube for the second and third rounds of panning. The procedures of the second and third rounds of panning were the same as the previous procedures.

### c. Preliminary screening

The above colonies were picked into a 96-well plate containing YT/Amp medium (Sangon Biotech, Cat#: A507016-0250) and grown at 37 °C for 3 h. IPTG was added to give a final concentration of 1 mM, and the mixture was induced overnight at 30 °C. The supernatant was then pelleted by centrifugation, and the supernatant was taken for ELISA assay.

Screening by ELISA assay: 96-well plates (Corning 9018) were coated with 1 µg/mL hROR1 ECD-his/hROR2 ECD-his/mROR1 ECD-his proteins prepared with PBS in Example 1a above and incubated at 4 °C overnight. The liquid was then discarded, and the plates were washed 3 times with PBST, blocked with 5% milk for 2 h at room temperature, and washed 3 times with PBST. The taken supernatant was added at 100 µL/well, and the plates were incubated at room temperature for 1 h, followed by washing 3 times with PBST. 100 µL/well of secondary antibody (Sigma, Cat#: A8592) was added, the plates were incubated at room temperature for 1 h, and then washed. TMB was then added to the plates at 100 µL/well and incubated for 15 min at room temperature. Thereafter, the reaction was terminated and data were read.

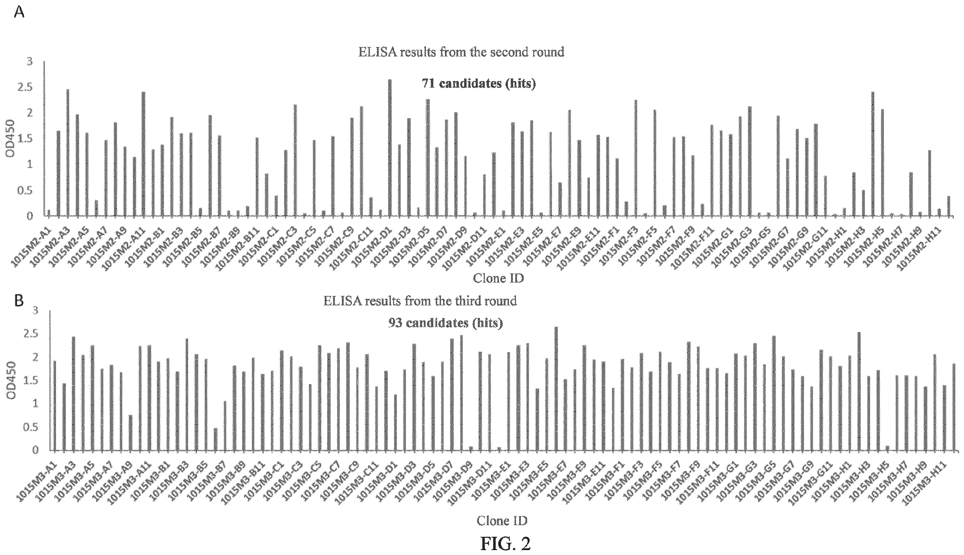

The results are shown in FIG. 2, which shows the ELISA results for 164 candidates (hits). Among them, 71 candidates were picked from the results of the second round of panning (as shown in A of FIG. 2) and 93 candidates were picked from the results of the third round of panning (as shown in B of FIG. 2).

### Example 3. Sequencing and FACS Screening Verification of Antibodies

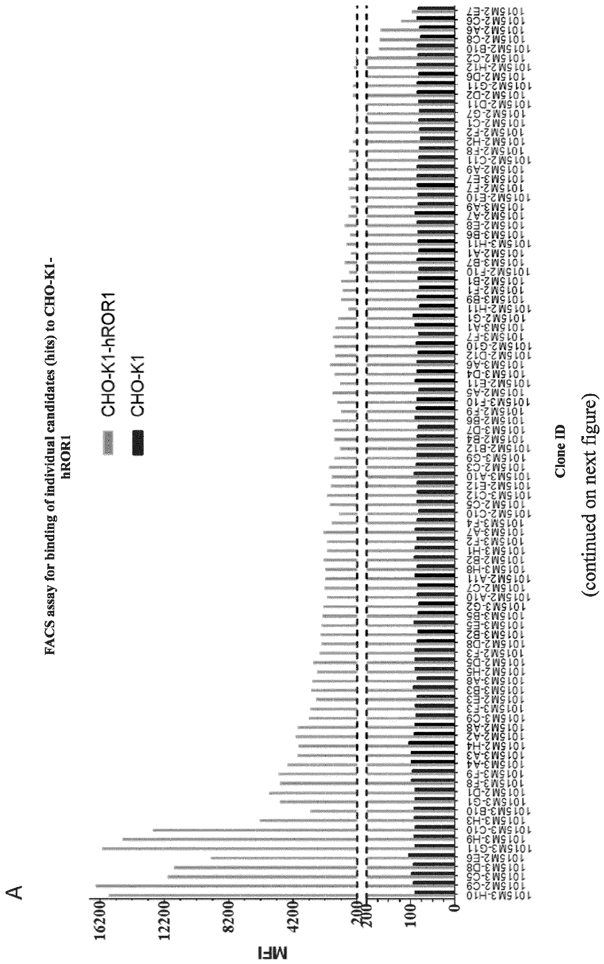

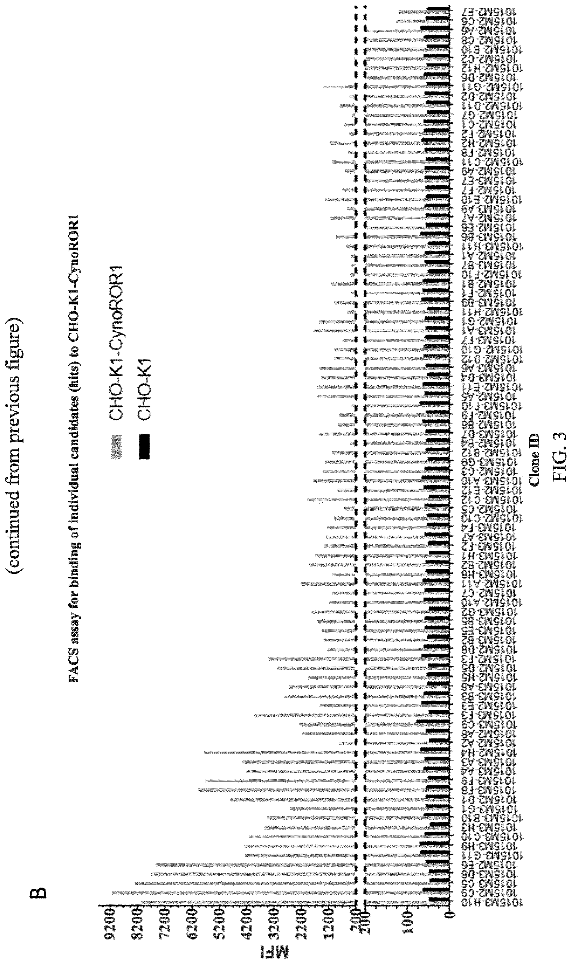

a. Sequencing: Phage-derived candidates: the above 164 candidates were directly sequenced (Tsingke Biotechnology Co., Ltd. (Beijing), Shanghai Branch), and then specific sequence analysis was performed. The results showed that a total of 89 unique candidates were generated.
b. FACS screening verification:
   The above 89 unique candidates were subjected to FACS screening experiments with HEK293T-hROR1 and CHO-K1-CynoROR1. The results are shown in A and B of FIG. 3, respectively. The FACS (flow cytometry) screening was conducted as follows: the above adherent cells were digested with TypLE, then washed with an FACS buffer and counted, followed by dilution to a density of (3-5)×10⁶/mL with blocking reagent. The cells were added to a 96-well plate (Corning 3894) at 100 µL/well. After blocking for 3-4 min, the supernatant described in Example 2c above was added at 100 µL/well, and the mixture was incubated for 1 h at 4 °C. After washing, the secondary antibody was added, and the mixture was incubated at 4 °C. The cells were then washed and subjected to flow cytometry for FACS analysis. The clones were ranked according to the results shown in FIG. 3, and the top 25 clones were paired for subsequent transfection and purification.
c. Sequence information of top 25 antibodies

It is well known to those skilled in the art that the CDRs of an antibody can be defined in the art using a variety of methods, such as the Kabat scheme based on sequence variability (see Kabat et al., Sequences of Proteins of Immunological Interest, Fifth Edition, National Institutes of Health (U.S.), Bethesda, Maryland (1991)), and the Chothia scheme based on the location of the structural loop regions (see J Mol Biol 273: 927-948, 1997). In the present application, the Combined scheme comprising the Kabat scheme and the Chothia scheme can also be used to determine the amino acid residues in a variable domain sequence. The Combined scheme combines the Kabat scheme with the Chothia scheme to obtain a larger range. See Table 3 for details.

**Table 3. Positions of CDRs of the present invention in antibody light or heavy chains based on different numbering schemes**

| CDR\Numbering scheme | Kabat | Chothia | Combined |
|---|---|---|---|
| VL CDR1 | L24 - L34 | L24 - L34 | L24 - L34 |
| VL CDR2 | L50 - L56 | L50 - L56 | L50 - L56 |
| VL CDR3 | L89 - L97 | L89 - L97 | L89 - L97 |
| VH CDR1 | H31 - H35 | H26 - H32 | H26 - H35 |
| VH CDR2 | H50 - H65 | H52 - H56 | H50 - H65 |
| VH CDR3 | H95 - H102 | H95 - H102 | H95 - H102 |

**Table 4. Amino acid sequences of heavy chain CDRs of the antibodies of the present invention (based on the Chothia scheme)**

| Antibody No. | VH CDR1 | **SEQ ID NO.** | VH CDR2 | **SEQ ID NO.** | VH CDR3 | **SEQ ID NO.** |
|---|---|---|---|---|---|---|
| Positive control (PR000374) | GSDINDY | 11 | NSGGS | 31 | GYSTYYRDFNI | 51 |
| PR001352 | GFTFSSY | 12 | WYDGIS | 32 | DLARSFDL | 52 |
| PR001353 | GFTFSSY | 12 | WYDGIS | 32 | EQARSFDL | 53 |
| PR001354 | GFTFSSY | 12 | WYDGSD | 33 | EQARSFDL | 53 |
| PR001355 | GFTFSSY | 12 | WYDGSN | 34 | EQARSFDL | 53 |
| PR001356 | GFTFSSY | 12 | WYDGVS | 35 | EQARSFDL | 53 |
| PR001357 | GFTFSSY | 12 | WYDGSN | 34 | EQARSFDL | 53 |
| PR001358 | GFTFSSY | 12 | WYDGSN | 34 | EQARSFDL | 53 |
| PR001359 | GFTFSTY | 13 | WYDGSD | 33 | EQARSFDL | 53 |
| PR001360 | GFTFSSY | 12 | WYDGSD | 33 | EQARSFDL | 53 |
| PR001361 | GFTFSRY | 14 | WYDGVS | 35 | EQARSFDL | 53 |
| PR001362 | GFTFSTY | 13 | WYDGSD | 33 | DQARSFDL | 54 |
| PR001363 | GFSFSSY | 15 | WYDGGD | 36 | EQARSFDL | 53 |
| PR001364 | GFTFSSF | 16 | WYDGSD | 33 | EQARSFDL | 53 |
| PR001365 | GFTFSSY | 12 | WYDGSD | 33 | EQARSFDL | 53 |
| PR001366 | GFTFSSY | 12 | WYDGIS | 32 | DLARSFDI | 55 |
| PR001367 | GFSFSSY | 15 | WYDGGD | 36 | EQARSFDL | 53 |
| PR001368 | GFTFSTY | 13 | WYDGSD | 33 | EQARSFDL | 53 |
| PR001369 | GFTFSNY | 17 | WYDGSN | 34 | EQARSFDL | 53 |
| PR001370 | GFTFSSY | 12 | WYDGIS | 32 | DLARSFDL | 52 |
| PR001371 | GFTFSSY | 12 | WYDGVS | 35 | EQARSFDL | 53 |
| PR001415 | GFTFSNS | 18 | WYDGSN | 34 | EQARSFDL | 53 |
| PR001416 | GFTFSTY | 13 | WYDGSD | 33 | EQARSFDL | 53 |
| PR001417 | GFSFSSY | 15 | WYDGGD | 36 | EQARSFDL | 53 |
| PR001418 | GFTFSSY | 12 | WYDGSD | 33 | EQARSFDL | 53 |
| PR001419 | GFTFSSY | 12 | WYDGIS | 32 | EQARSFDL | 53 |

**Table 5. Amino acid sequences of light chain CDRs of the antibodies of the present invention (based on the Chothia scheme)**

| Antibody No. | VL CDR1 | **SEQ ID NO.** | VL CDR2 | **SEQ ID NO.** | VL CDR3 | **SEQ ID NO.** |
|---|---|---|---|---|---|---|
| Positive control antibody (PR000374) | QASQSIDSNLA | 68 | RASNLAS | 77 | LGGVGAVSYRTS | 83 |
| PR001352 | RASQTINNWLA | 69 | KASSLES | 78 | QQYNSYSPMYT | 84 |
| PR001353 | RASQSISSWLA | 70 | KASSLES | 78 | QQYKSYSPMYT | 85 |
| PR001354 | RASQTINNWLA | 69 | KASSLES | 78 | QQYNSYSPMYT | 84 |
| PR001355 | RASQTMNNWLA | 71 | KASSLES | 78 | QQYNSYSPMYT | 84 |
| PR001356 | RASQSINNWLA | 72 | KASSLES | 78 | QQYKSYSPMYT | 85 |
| PR001357 | RASQTINNWLA | 69 | KASSLES | 78 | QQYKLYSPMYT | 86 |
| PR001358 | RASQSINSWLA | 73 | KASNLES | 79 | QQYKSYSPMYT | 85 |
| PR001359 | RASQSVNNWLA | 74 | KASSLES | 78 | QQYKSYSPMYT | 85 |
| PR001360 | RASQTINNWLA | 69 | KASSLES | 78 | QQYNIYSPMYT | 87 |
| PR001361 | RASQSINSWLA | 73 | KASSLES | 78 | QQYKSYSPMYT | 85 |
| PR001362 | RASQSINSWLA | 73 | KASSLES | 78 | QQYKSYSPMYT | 85 |
| PR001363 | RASQTINNWLA | 69 | KASSLES | 78 | QQYNSYSPMYT | 84 |
| PR001364 | RASQTINNWLA | 69 | KASSLES | 78 | QQYNSYSPMYT | 84 |
| PR001365 | RASQTINNWLA | 69 | KASSLES | 78 | QQYKLYSPMYT | 86 |
| PR001366 | RASQSINSWLA | 73 | KASSLES | 78 | QQYKSYSPMYT | 85 |
| PR001367 | RASQTINNWLA | 69 | KASSLES | 78 | QQYNSYSPMYT | 84 |
| PR001368 | RASQSINSWLA | 73 | KASSLES | 78 | QQYKSYSPMYT | 85 |
| PR001369 | RASQTINNWLA | 69 | KASSLES | 78 | QQYNSYSPMYT | 84 |
| PR001370 | RASQSINNWLA | 72 | KASSLES | 78 | QQYNSYSPMYT | 84 |
| PR001371 | RASQTINNWLA | 69 | KASSLES | 78 | QQYNSYSPMYT | 84 |
| PR001415 | RASQTINNWLA | 69 | KASSLES | 78 | QQYNSYSPMYT | 84 |
| PR001416 | RASQTINNWLA | 69 | KASSLES | 78 | QQYNSYSPMYT | 84 |
| PR001417 | RASQSINSWLA | 73 | KASSLES | 78 | QQYNSYSPMYT | 84 |
| PR001418 | RASQTINNWLA | 69 | KASSLES | 78 | QQYNSYSPMYT | 84 |
| PR001419 | RASQSISSWLA | 70 | KASSLES | 78 | QQYNSYSPMYT | 84 |

For specific information of the positive control antibody (PR000374), see WO2016094873 (R11H18L9).

### d. Sequences of framework regions of antibody variable regions

Table 5-1 below shows the combination of sequences of the preferred framework regions of the control antibody and the antibodies of the present invention. The light and the heavy chain variable regions of the antibodies obtained in the present invention and the control antibody are shown in Table 5-1 below.

### e. Sequences of light and heavy chains of antibodies

After obtaining the sequences of light and heavy chain variable regions encoding the antibody molecules, the sequences of the light and heavy chain variable regions can be fused with the corresponding sequences of the light and heavy chain constant regions of the human antibody and expressed by conventional recombinant DNA techniques to obtain recombinant antibody molecules. The heavy chain constant region of the antibody of the present invention may be one of hIgG 1, hIgG2, hIgG3 and hIgG4. In this example, the sequence of the heavy chain variable region (VH) of the antibody was genetically synthesized and cloned into a mammalian cell expression plasmid vector encoding the sequence of the heavy chain constant region of the human IgG1 antibody to encode a full-length heavy chain producing the IgG1 antibody. The sequence of the light chain variable region (VL) of the antibody was genetically synthesized and cloned into a mammalian cell expression plasmid vector encoding the sequence of the κ light chain constant region of the human antibody to encode a full-length light chain producing the antibody. In this example, the sequences of the variable regions of the monoclonal antibody molecules obtained from immunized Harbour H2L2 mice were human antibody sequences, therefore, a fully human anti-ROR1 recombinant IgG1 antibody was also obtained from this example.

For example, the sequences of the heavy and light chains of the antibodies obtained in the present invention and the control antibody may be as shown in Table 5-1 below.

**Table 5-1. Combination of sequences of light and heavy chains, variable regions and framework regions (SEQ ID NO, the present invention takes the Chothia scheme as an example in the follow-up)**

| Accession | ChainType | Variable region | Light and heavy chain | FWR1 | FWR2 | FWR3 | FWR4 |
|---|---|---|---|---|---|---|---|
| PR001352 | HC (heavy chain) | 93 | 136 | 2 | 20 | 38 | 57 |
| PR001352 | LC (light chain) | 117 | 160 | 59 | 76 | 81 | 89 |
| PR001353 | HC | 94 | 137 | 3 | 21 | 39 | 57 |
| PR001353 | LC | 118 | 161 | 60 | 76 | 81 | 89 |
| PR001354 | HC | 95 | 138 | 4 | 22 | 40 | 57 |
| PR001354 | LC | 119 | 162 | 61 | 76 | 81 | 89 |
| PR001355 | HC | 96 | 139 | 5 | 23 | 41 | 57 |
| PR001355 | LC | 120 | 163 | 62 | 76 | 82 | 90 |
| PR001356 | HC | 97 | 140 | 6 | 24 | 42 | 57 |
| PR001356 | LC | 121 | 164 | 60 | 76 | 81 | 89 |
| PR001357 | HC | 98 | 141 | 4 | 23 | 40 | 57 |
| PR001357 | LC | 122 | 165 | 63 | 76 | 81 | 89 |
| PR001358 | HC | 99 | 142 | 4 | 25 | 43 | 57 |
| PR001358 | LC | 123 | 166 | 60 | 76 | 81 | 91 |
| PR001359 | HC | 100 | 143 | 6 | 26 | 40 | 57 |
| PR001359 | LC | 124 | 167 | 60 | 76 | 81 | 89 |
| PR001360 | HC | 101 | 144 | 6 | 23 | 44 | 57 |
| PR001360 | LC | 125 | 168 | 64 | 76 | 81 | 89 |
| PR001361 | HC | 102 | 145 | 7 | 27 | 45 | 57 |
| PR001361 | LC | 126 | 169 | 65 | 76 | 81 | 91 |
| PR001362 | HC | 103 | 146 | 8 | 23 | 46 | 57 |
| PR001362 | LC | 127 | 170 | 60 | 76 | 81 | 89 |
| PR001363 | HC | 104 | 147 | 6 | 28 | 47 | 57 |
| PR001363 | LC | 128 | 171 | 66 | 76 | 81 | 89 |
| PR001364 | HC | 105 | 148 | 2 | 23 | 40 | 57 |
| PR001364 | LC | 129 | 172 | 60 | 76 | 81 | 89 |
| PR001365 | HC | 106 | 149 | 4 | 23 | 40 | 57 |
| PR001365 | LC | 130 | 173 | 67 | 76 | 81 | 89 |
| PR001366 | HC | 107 | 150 | 9 | 20 | 38 | 57 |
| PR001366 | LC | 126 | 169 | 65 | 76 | 81 | 91 |
| PR001367 | HC | 108 | 151 | 6 | 28 | 48 | 57 |
| PR001367 | LC | 128 | 171 | 66 | 76 | 81 | 89 |
| PR001368 | HC | 109 | 152 | 6 | 23 | 49 | 57 |
| PR001368 | LC | 127 | 170 | 60 | 76 | 81 | 89 |
| PR001369 | HC | 110 | 153 | 6 | 29 | 43 | 57 |
| PR001369 | LC | 129 | 172 | 60 | 76 | 81 | 89 |
| PR001370 | HC | 93 | 136 | 2 | 20 | 38 | 57 |
| PR001370 | LC | 131 | 174 | 60 | 76 | 81 | 91 |
| PR001371 | HC | 111 | 154 | 6 | 27 | 45 | 57 |
| PR001371 | LC | 117 | 160 | 59 | 76 | 81 | 89 |
| PR001415 | HC | 112 | 155 | 10 | 22 | 40 | 57 |
| PR001415 | LC | 129 | 172 | 60 | 76 | 81 | 89 |
| PR001416 | HC | 113 | 156 | 6 | 23 | 40 | 57 |
| PR001416 | LC | 129 | 172 | 60 | 76 | 81 | 89 |
| PR001417 | HC | 108 | 151 | 6 | 28 | 48 | 57 |
| PR001417 | LC | 132 | 175 | 66 | 76 | 81 | 89 |
| PR001418 | HC | 114 | 157 | 6 | 23 | 40 | 57 |
| PR001418 | LC | 133 | 176 | 60 | 76 | 81 | 90 |
| PR001419 | HC | 115 | 158 | 6 | 30 | 50 | 57 |
| PR001419 | LC | 134 | 177 | 65 | 76 | 81 | 89 |
| Positive control (PR000374) | | 92 | 135 | | | | |
| Positive control (PR000374) | | 116 | 159 | | | | |

### Example 4. Production, Purification and Verification of Antibodies

### a. Production and purification of recombinant antibodies

After obtaining the sequences of light and heavy chain variable regions encoding the antibody molecules through the above sequencing analysis, the heavy and light chains were paired and cloned into an expression vector by conventional recombinant DNA techniques. The top 25 clones described above were paired, and then transfected and purified. Proteins were then produced by transfection into 293 or CHO cells. The antibodies were purified using protein A and then characterized by conventional procedures in the art. The specific results are shown in Table 6.

**Table 6**

| **AccessionID** | **AB_M W(Kda)** | **Ext. Coeff** | **AB_pI** | **Yield (mg/L)** | **SEC purificati on** | **VH germline** | **VL germline** | **VH PTMs** | **VL PTMs** |
|---|---|---|---|---|---|---|---|---|---|
| PR001352 | 145.3 | 1.45 | 9.28 | 26.86 | 100 | IGHV3-33 | IGKV1-5 | DG (HCDR2) | NS (LCDR3) |
| PR001353 | 145.174 | 1.45 | 9.28 | 38.06 | 99.49 | | | DG (HCDR2) | |
| PR001354 | 145.316 | 1.46 | 9.27 | 35.64 | 100 | | | DG (HCDR2) | NS (LCDR3) |
| PR001355 | 145.138 | 1.47 | 9.27 | 39 | 100 | | | DG (HCDR2) | NS (LCDR3) |
| PR001356 | 145.162 | 1.47 | 9.17 | 42.84 | 99 | | | DG (HCDR2) | |
| PR001357 | 145.504 | 1.48 | 9.42 | 33.66 | 100 | | | DG (HCDR2) | |
| PR001358 | 145.256 | 1.45 | 9.37 | 34.46 | 100 | | | DG (HCDR2) | NS (LCDR1) |
| PR001359 | 145.258 | 1.46 | 9.27 | 40.99 | 99.56 | | | DG (HCDR2) | |
| PR001360 | 145.324 | 1.45 | 9.05 | 30.92 | 100 | | | DG (HCDR2) | |
| PR001361 | 145.136 | 1.45 | 9.29 | 36.69 | 98.91 | | | DG (HCDR2) | NS (LCDR1) |
| PR001362 | 145.086 | 1.47 | 8.87 | 33.8 | 99.29 | | | DG (HCDR2) | NS (LCDR1) |
| PR001363 | 145.212 | 1.45 | 8.64 | 44.43 | 99.38 | | | DG (HCDR2) | NS (LCDR3) |
| PR001364 | 145.314 | 1.45 | 9.05 | 40.06 | 99.35 | | | DG (HCDR2) | NS (LCDR3) |
| PR001365 | 145.542 | 1.48 | 9.34 | 21.82 | 100 | | | DG (HCDR2) | |
| PR001366 | 145.242 | 1.45 | 9.44 | 47.01 | 100 | | | DG (HCDR2) | NS (LCDR1) |
| PR001367 | 145.364 | 1.46 | 8.63 | 38.08 | 99.06 | | | DG (HCDR2) | NS (LCDR3) |
| PR001368 | 145.202 | 1.46 | 9.17 | 44.64 | 98.47 | | | DG (HCDR2) | NS (LCDR1) |
| PR001369 | 145.276 | 1.45 | 9.18 | 38.9 | 99.44 | | | DG (HCDR2) | NS (LCDR3) |
| PR001370 | 145.25 | 1.45 | 9.28 | 28.5 | 99.7 | | | DG (HCDR2) | NS (LCDR3) |
| PR001371 | 144.993 | 1.45 | 8.88 | 41.52 | 99.11 | | | DG (HCDR2) | NS (LCDR3) |
| PR001415 | 145.282 | 1.45 | 9.18 | 89.5 | 96.78 | | | NS (HCDR1),DG (HCDR2) | NS (LCDR3) |
| PR001416 | 145.31 | 1.46 | 9.17 | 94.25 | 97.35 | | | DG (HCDR2) | NS (LCDR3) |
| PR001417 | 145.282 | 1.46 | 8.63 | 94.5 | 96.45 | | | DG (HCDR2) | NS (LCDR1),NS (LCDR3) |
| PR001418 | 145.31 | 1.46 | 9.17 | 74.5 | 97.17 | | | DG (HCDR2) | NS (LCDR3) |
| PR001419 | 145.138 | 1.47 | 9.27 | 86 | 98.12 | | | DG (HCDR2),NxS/T (HFR3) | NS (LCDR3) |

### b. Verification of recombinant antibodies

### (1) Verification of antibody-binding properties

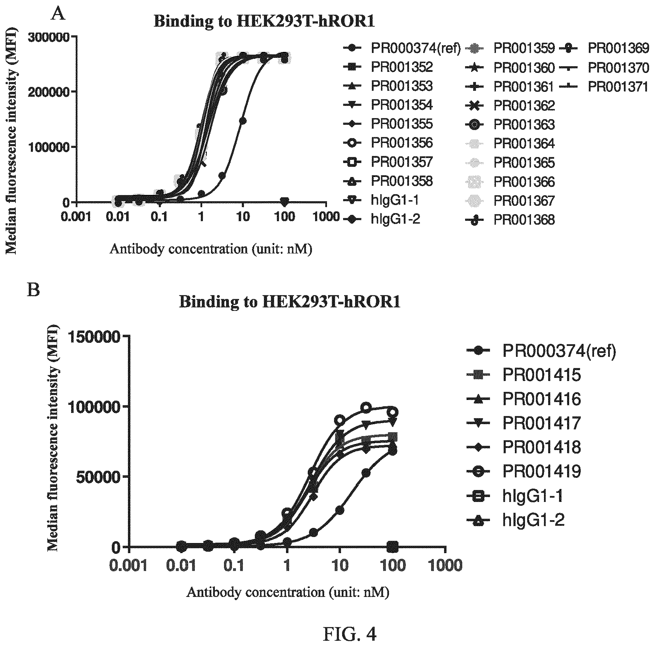

The binding properties were characterized by FACS. The recombinant antibodies obtained above were serially diluted to 9 different concentrations and incubated for 1 h, and then determined for the binding ability with HEK293T-hROR1, CHO-K1-cynoROR1 and A549 cell lines (endogenously expressing ROR1), respectively. The secondary antibody was then incubated for 1 h. The results are shown in FIG. 4-6 and Table 7-2 below.

**Table 7-1**

| | HEK293T-hROR1 | | CHO-K1-cynoROR1 | | A549 | |
|---|---|---|---|---|---|---|
| Ab No. | EC50 | MAX(MFI) | EC50 | MAX(MFI) | EC50 | MAX(MFI) |
| PR000374 | 8.776 | 261573 | 3.449 | 31869 | 3.581 | 3846 |
| PR001352 | 1.127 | 261573 | 0.4629 | 30743 | 0.1542 | 4520 |
| PR001353 | 1.031 | 261573 | 0.3096 | 29027 | 0.1019 | 4331 |
| PR001354 | 1.135 | 261573 | 0.4362 | 26956 | 0.1852 | 4305 |
| PR001355 | 1.137 | 261573 | 0.4629 | 30016 | 0.1767 | 4240 |
| PR001356 | 1.016 | 261573 | 0.4831 | 29730 | 0.1658 | 4631 |
| PR001357 | 0.9739 | 261573 | 0.3244 | 24566 | 0.09886 | 4548 |
| PR001358 | 1.031 | 261573 | 0.3388 | 27345 | 0.08687 | 4968 |
| PR001359 | 1.134 | 261573 | 0.5516 | 27150 | 0.184 | 3929 |
| PR001360 | 1.443 | 261573 | 0.6241 | 24978 | 0.2394 | 4051 |
| PR001361 | 1.187 | 261573 | 0.4089 | 27215 | 0.1287 | 4520 |
| PR001362 | 1.685 | 261573 | 1.947 | 17654 | 18.61 | 3754 |
| PR001363 | 1.567 | 261573 | 0.5852 | 17205 | 0.2057 | 3697 |
| PR001364 | 1.523 | 261573 | 0.4849 | 18158 | 0.1955 | 3965 |
| PR001365 | 1.149 | 261573 | 0.4081 | 22079 | 0.1323 | 4398 |
| PR001366 | 1.06 | 261573 | 0.2652 | 23316 | 0.1126 | 4746 |
| PR001367 | 1.239 | 261573 | 0.4939 | 21210 | 0.2049 | 3447 |
| PR001368 | 1.076 | 261573 | 0.4835 | 27806 | 0.1938 | 4214 |
| PR001369 | 1.469 | 261573 | 0.4474 | 22184 | 0.1344 | 3893 |
| PR001370 | 1.276 | 261573 | 0.4838 | 26010 | 0.1298 | 3917 |
| PR001371 | 1.318 | 261573 | 0.7295 | 28410 | 0.2551 | 4113 |
| hIgG1 | | 226 | | 140 | | 119 |

**Table 7-2**

| | HEK293T-hROR1 | CHO-K1-cyROR1 | A549 |
|---|---|---|---|
| Ab No. | EC50 | EC50 | EC50 |
| PR000374 | 17.21 | 5.044 | 6.337 |
| PR001415 | 2.522 | 0.8506 | 0.3761 |
| PR001416 | 2.395 | 0.9467 | 0.3978 |
| PR001417 | 2.831 | 1.491 | 0.9127 |
| PR001418 | 3.005 | 1.105 | 0.5006 |
| PR001419 | 2.679 | 0.7063 | 0.2472 |

FIG. 4 illustrates the ELISA assays on the binding of recombinant antibodies to a 293T-hROR1 cell line; wherein, A shows the binding of PR001352-PR001371, and that the EC₅₀ data of all 20 recombinant antibodies (see Table 7-1 for the EC₅₀ data, wherein the data in column 3 (MAX (MFI)) were exactly the same due to too high expression of positive cells and thus complete saturation of the flow cytometer) were 10-fold lower than that of the reference antibody (PR000374, R11H18L9 from patent WO2016094873) in the prior art; and B shows that the EC₅₀ data of PR001415-1419 (see Table 7-2 for the EC₅₀ data) were also 10-fold lower than that of the reference antibody in the prior art; wherein iso1 and iso2 were both hIgG1, purchased from Crownbio, Cat#: C0001-4.

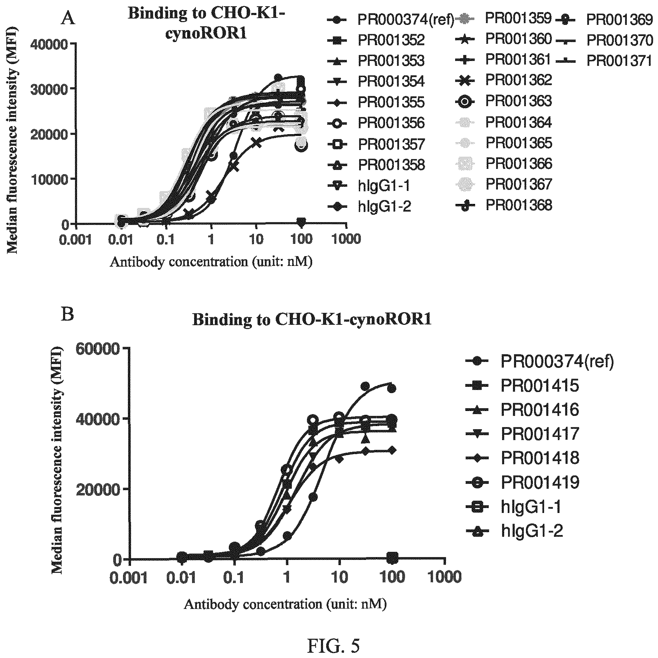

FIG. 5 illustrates the ELISA assays on the binding of recombinant antibodies to a CHO-K1-cynoROR1 cell line; wherein, A shows the binding data of PR001352-PR001371 (see Table 7-1 for the EC₅₀ data), demonstrating that these recombinant antibodies all had cross-reactivity with cynoROR1, and except for PR001362, all had binding effect superior to that of the reference antibody (PR000374) in the prior art; and B shows the binding data of PR001415-PR001419 (see Table 7-2 for the EC₅₀ data), demonstrating that these recombinant antibodies all had cross-reactivity with cynoROR1 which is superior to the reference antibody in the prior art.

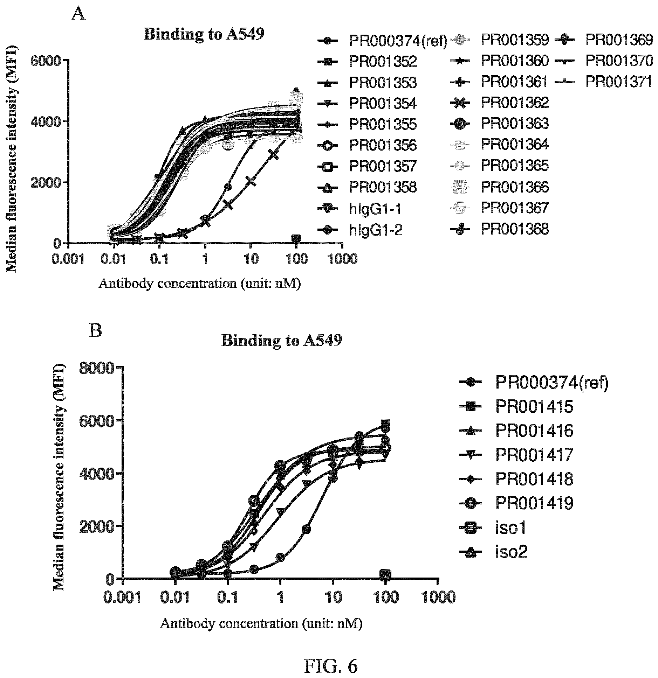

FIG. 6 illustrates the ELISA assays on the binding of recombinant antibodies to an A549 cell line; wherein, A shows the binding data of PR001352-PR001371, and the results (see Table 7-1 for the EC₅₀ data) show that PR001352-PR001371, except for PR001362, were superior to the reference antibody (PR000374) in the prior art; and B shows the binding data of PR001415-PR001419 (see Table 7-2 for the EC₅₀ data), and the results show that all 5 recombinant antibodies had better affinity than the reference antibody in the prior art.

### (2) Endocytosis verification

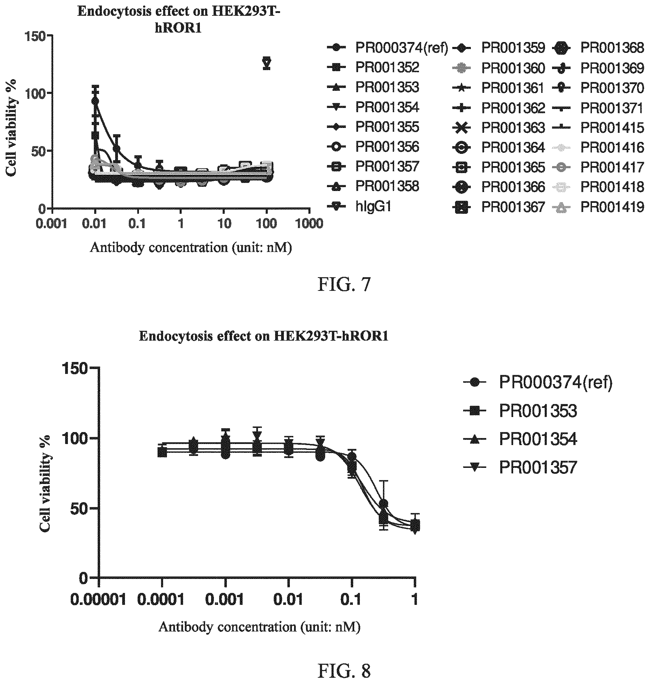

For endocytosis experiments, HEK293T-hROR1 cells were counted and seeded in black-wall and transparent-bottom 96-well plates (PE 6005225) at 5000 cells/well. The recombinant antibodies obtained above were serially diluted to 9 different concentrations, and the initial concentration of the final concentrations of the recombinant antibodies after addition to the cells was 100 nM. a-hFc-MMAF (purchased from Moradec LLC, Cat#: AH-102AF-50) was added to give a final concentration of 1 µg/mL. Thereafter, the plates were incubated at 37 °C with 5% CO₂ for 72 h, and then the cells were lysed with a CTG kit (purchased from Promega, Cat#: G7573) and the luminescence was detected by an Enspire instrument. The results are shown in FIG. 7 and 8 and Table 8 below.

FIG. 7 illustrates the data from endocytosis experiments of PR001352-PR001371 and PR001415-PR001419 on HEK293T-hROR1. It can be seen from the figure that all recombinant antibodies showed a very good internalization effect, and even the antibody with the lowest concentration achieved complete endocytosis effect.

FIG. 8 shows the data from endocytosis experiments of PR001353, PR001354 and PR001357 at lower concentrations on HEK293T-hROR1 cells.

**Table 8**

| Ab ID | EC50(nM) |
|---|---|
| PR000374(ref) | 0.2429 |
| PR001353 | 0.1489 |
| PR001354 | 0.1428 |
| PR001357 | 0.1371 |

### (3) Determination of KD values

The KD values of the above antibodies were determined using conventional procedures in the art. Table 9 shows a KD summary of PR001352-PR001371 and PR001415-PR1419. These results show that the effects of most antibodies were comparable to that of the reference antibody in the prior art, and 6 of these antibodies were picked for subsequent ADCC/CDC/ADCP assays.

**Table 9**

| **Sample ID** | **KD (M)** | **kon(1/Ms)** | **kdis(1/s)** |
|---|---|---|---|
| **PR001368** | 2.16E-09 | 4.02E+05 | 8.67E-04 |
| **PR001369** | 1.69E-09 | 3.07E+05 | 5.19E-04 |
| **PR001370** | 3.97E-09 | 2.85E+05 | 1.13E-03 |
| **PR001371** | 1.76E-09 | 2.98E+05 | 5.25E-04 |
| **PR001415** | 1.53E-09 | 3.36E+05 | 5.12E-04 |
| **PR001416** | 1.27E-09 | 2.70E+05 | 3.42E-04 |
| **PR001417** | 1.49E-08 | 4.33E+05 | 6.46E-03 |
| **PR001418** | 1.45E-09 | 2.92E+05 | 4.23E-04 |
| **PR001419** | 3.01E-09 | 3.89E+05 | 1.17E-03 |
| **PR000374** | 3.97E-10 | 4.37E+05 | 1.73E-04 |
| **PR001352** | 1.51E-09 | 3.16E+05 | 4.76E-04 |
| **PR001353** | 2.21E-09 | 4.19E+05 | 9.27E-04 |
| **PR001354** | 1.13E-09 | 3.39E+05 | 3.83E-04 |
| **PR001355** | 8.39E-10 | 3.47E+05 | 2.91E-04 |
| **PR001356** | 8.10E-10 | 3.54E+05 | 2.87E-04 |
| **PR001357** | 4.81E-10 | 3.97E+05 | 1.91E-04 |
| **PR001358** | 1.07E-09 | 4.82E+05 | 5.18E-04 |
| **PR001359** | 2.00E-09 | 3.20E+05 | 6.37E-04 |
| **PR001360** | 8.04E-10 | 3.09E+05 | 2.48E-04 |
| **PR001361** | 1.83E-09 | 5.90E+05 | 1.08E-03 |
| **PR001362** | 4.54E-08 | 3.00E+05 | 1.36E-02 |
| **PR001363** | 2.90E-09 | 3.48E+05 | 1.01E-03 |
| **PR001364** | 1.20E-09 | 3.14E+05 | 3.76E-04 |
| **PR001365** | 2.37E-10 | 3.25E+05 | 7.72E-05 |
| **PR001366** | 1.67E-09 | 4.48E+05 | 7.50E-04 |
| **PR001367** | 3.06E-09 | 3.41E+05 | 1.04E-03 |

### c. PTM (post-translational modification) removal experiments

Based on the binding data, four primary clones PR001354, PR001356, PR001364 and PR001369 were selected for PTM removal experiments. Generally, the PTM removal strategy is as shown in Tables 10-13 below. NG was mutated to NA or QG; NS was mutated to NA or QS; and DG was mutated to EG or DA. The sequence information of the variants is shown in Table 13-1.

**Table 10. List of PR001354 variants**

| **Variant** | **Mutant VH** | **Mutant VL** |
|---|---|---|
| PR001354(WT) | PR001354_VH | PR001354_VL |
| PR002562 | PR001354_VH | PR001354_VL_N92Q |
| PR002563 | PR001354_VH | PR001354_VL_S93A |
| PR002564 | PR001354_VH_D54E | PR001354_VL |
| PR002565 | PR001354_VH_D54E | PR001354_VL_N92Q |
| PR002566 | PR001354_VH_D54E | PR001354_VL_S93A |
| PR002567 | PR001354_VH_G55A | PR001354_VL |
| PR002568 | PR001354_VH_G55A | PR001354_VL_N92Q |
| PR002569 | PR001354_VH_G55A | PR001354_VL_S93A |

**Table 11. List of PR001356 variants**

| **Variant** | **Mutant VH** | **Mutant VL** |
|---|---|---|
| PR001356(WT) | PR001356_VH(WT) | PR001356_VL(WT) |
| PR002570 | PR001356_VH_D54E | PR001356_VL |
| PR002571 | PR001356_VH_G55A | PR001356_VL |

**Table 12. List of PR001364 variants**

| **Variant** | **Mutant VH** | **Mutant VL** |
|---|---|---|
| PR001364(WT) | PR001364_VH | PR001364_VL |
| PR002572 | PR001364_VH | PR001364_VL_N92Q |
| PR002573 | PR001364_VH | PR001364_VL_S93A |
| PR002574 | PR001364_VH_D54E | PR001364_VL |
| PR002575 | PR001364_VH_D54E | PR001364_VL_N92Q |
| PR002576 | PR001364_VH_D54E | PR001364_VL_S93A |
| PR002577 | PR001364_VH_G55A | PR001364_VL |
| PR002578 | PR001364_VH_G55A | PR001364_VL_N92Q |
| PR002579 | PR001364_VH_G55A | PR001364_VL_S93A |

**Table 13. List of PR001369 variants**

| **Variant** | **Mutant VH** | **Mutant VL** |
|---|---|---|
| PR001369(WT) | PR001369_VH | PR001369_VL |
| PR002580 | PR001369_VH | PR001369_VL_N92Q |
| PR002581 | PR001369_VH | PR001369_VL_S93A |
| PR002582 | PR001369_VH_D54E | PR001369_VL |
| PR002583 | PR001369_VH_D54E | PR001369_VL_N92Q |
| PR002584 | PR001369_VH_D54E | PR001369_VL_S93A |
| PR002585 | PR001369_VH_G55A | PR001369_VL |
| PR002586 | PR001369_VH_G55A | PR001369_VL_N92Q |
| PR002587 | PR001369_VH_G55A | PR001369_VL_S93A |

**Table 13-1. Sequence information of PR001354, PR001356, PR001364 and PR001369 variants**

| **Antibody No.** | **Light chain** | **Heavy chain** | **VL** | **VH** | **LCDR 1** | **LCDR 2** | **LCDR 3** | **HCDR 1** | **HCDR 2** | **HCDR 3** |
|---|---|---|---|---|---|---|---|---|---|---|
| PR002562 | 206 | 138 | 194 | 95 | 69 | 78 | 184 | 12 | 33 | 53 |
| PR002563 | 207 | 138 | 195 | 95 | 69 | 78 | 185 | 12 | 33 | 53 |
| PR002564 | 162 | 198 | 119 | 186 | 69 | 78 | 84 | 12 | 178 | 53 |
| PR002565 | 206 | 198 | 194 | 186 | 69 | 78 | 184 | 12 | 178 | 53 |
| PR002566 | 207 | 198 | 195 | 186 | 69 | 78 | 185 | 12 | 178 | 53 |
| PR002567 | 162 | 199 | 119 | 187 | 69 | 78 | 84 | 12 | 179 | 53 |
| PR002568 | 206 | 199 | 194 | 187 | 69 | 78 | 184 | 12 | 179 | 53 |
| PR002569 | 207 | 199 | 195 | 187 | 69 | 78 | 185 | 12 | 179 | 53 |
| PR002570 | 164 | 200 | 121 | 188 | 72 | 78 | 85 | 12 | 180 | 53 |
| PR002571 | 164 | 201 | 121 | 189 | 72 | 78 | 85 | 12 | 181 | 53 |
| PR002572 | 208 | 148 | 196 | 105 | 69 | 78 | 184 | 16 | 33 | 53 |
| PR002573 | 209 | 148 | 197 | 105 | 69 | 78 | 185 | 16 | 33 | 53 |
| PR002574 | 172 | 202 | 129 | 190 | 69 | 78 | 84 | 16 | 178 | 53 |
| PR002575 | 208 | 202 | 196 | 190 | 69 | 78 | 184 | 16 | 178 | 53 |
| PR002576 | 209 | 202 | 197 | 190 | 69 | 78 | 185 | 16 | 178 | 53 |
| PR002577 | 172 | 203 | 129 | 191 | 69 | 78 | 84 | 16 | 179 | 53 |
| PR002578 | 208 | 203 | 196 | 191 | 69 | 78 | 184 | 16 | 179 | 53 |
| PR002579 | 209 | 203 | 197 | 191 | 69 | 78 | 185 | 16 | 179 | 53 |
| PR002580 | 208 | 153 | 196 | 110 | 69 | 78 | 184 | 17 | 34 | 53 |
| PR002581 | 209 | 153 | 197 | 110 | 69 | 78 | 185 | 17 | 34 | 53 |
| PR002582 | 172 | 204 | 129 | 192 | 69 | 78 | 84 | 17 | 182 | 53 |
| PR002583 | 208 | 204 | 196 | 192 | 69 | 78 | 184 | 17 | 182 | 53 |
| PR002584 | 209 | 204 | 197 | 192 | 69 | 78 | 185 | 17 | 182 | 53 |
| PR002585 | 172 | 205 | 129 | 193 | 69 | 78 | 84 | 17 | 183 | 53 |
| PR002586 | 208 | 205 | 196 | 193 | 69 | 78 | 184 | 17 | 183 | 53 |
| PR002587 | 209 | 205 | 197 | 193 | 69 | 78 | 185 | 17 | 183 | 53 |

The results are shown in Table 14, which shows the PTM removal design and characteristic data for PR001354, PR001356, PR001364 and PR001369. It can be seen from the table that the removal mutations at most of the PTM sites do not affect the binding ability of the antibody molecules, which is more conducive to the downstream production of patent medicines.

**Table 14**

| **Antibody No.** | **Description of structures** | **Dissociation rate measured by Octet** | | **Panc-1 binding (MFI)** | **HEK293T-hROR1 binding (MFI)** |
|---|---|---|---|---|---|
| | | Response | kdis(1/s) | | |
| **PR001354** | anti-ROR1 1015M2-C9 hIgG1 | 0.6087 | 1.17E-04 | 6703 | 42753 |
| PR002562 | anti-ROR1 1015M2-C9(L:N92Q) hIgG1 | 0.6286 | 1.62E-04 | 5604 | 43906 |
| PR002563 | anti-ROR1 1015M2-C9(L:S93A) hIgG1 | 0.7569 | 1.22E-04 | 5018 | 43800 |
| PR002564 | anti-ROR1 1015M2-C9(H:D54E) hIgG1 | 0.7481 | 1.08E-04 | 6588 | 43169 |
| PR002565 | anti-ROR1 1015M2-C9(H:D54E; L:N92Q) hIgG1 | 0.5949 | 1.06E-04 | 6821 | 46872 |
| PR002566 | anti-ROR1 1015M2-C9(H:D54E; L:S93A) hIgG1 | 0.6635 | 1.35E-04 | 4633 | 43273 |
| PR002567 | anti-ROR1 1015M2-C9(H:G55A) hIgGl | 0.7074 | 2.15E-04 | 7033 | 41934 |
| PR002568 | anti-ROR1 1015M2-C9(H:G55A; L:N92Q) hIgGl | 0.7024 | 1.40E-04 | 3619 | 46307 |
| PR002569 | anti-ROR1 1015M2-C9(H:G55A; L:S93A) hIgG1 | 0.6139 | 2.07E-04 | 3761 | 34497 |
| | | | | | |
| **PR001356** | anti-ROR1 1015M2-E6 hIgG1 | 0.6173 | 1.52E-04 | 5592 | 50900 |
| PR002570 | anti-ROR1 1015M2-E6(H:D54E) hIgG1 | 0.4717 | 1.08E-03 | 3656 | 43064 |
| PR002571 | anti-ROR1 1015M2-E6(H:G55A) hIgG1 | 0.6383 | 1.13E-04 | 4556 | 49559 |
| | | | | | |
| **PR001364** | anti-ROR1 1015M3-D8 hIgG1 | 0.6014 | 9.38E-05 | 4965 | 43064 |
| PR002572 | anti-ROR1 1015M3-D8(L:N92Q) hIgG1 | 0.6057 | 1.04E-04 | 3926 | 42960 |
| PR002573 | anti-ROR1 1015M3-D8(L:S93A) hIgG1 | 0.7228 | 8.84E-05 | 3708 | 37169 |
| PR002574 | anti-ROR1 1015M3-D8(H:D54E) hIgG1 | 0.4852 | 1.72E-04 | 5874 | 45309 |
| PR002575 | anti-ROR1 1015M3-D8(H:D54E; L:N92Q) hIgG1 | 0.8283 | 1.12E-04 | 3253 | 33759 |
| PR002576 | anti-ROR1 1015M3-D8(H:D54E; L:S93A) hIgG1 | 0.8656 | 9.83E-05 | 2466 | 40247 |
| PR002577 | anti-ROR1 1015M3-D8(H:G55A) hIgG1 | 0.2442 | 6.01E-04 | 6488 | 44226 |
| PR002578 | anti-ROR1 1015M3-D8(H:G55A; L:N92Q) hIgG1 | 0.4142 | 3.22E-04 | 6703 | 45090 |
| PR002579 | anti-ROR1 1015M3-D8(H:GSSA; L:S93A) hIgG1 | 0.7828 | 1.99E-04 | 7049 | 43064 |
| | | | | | |
| **PR001369** | anti-ROR1 1015M3-H10 hIgG1 | 0.2883 | 4.98E-03 | 6226 | 43800 |
| PR002580 | anti-ROR1 1015M3-H10(L:N92Q) hIgG1 | 0.9847 | 4.75E-05 | 3716 | 42547 |
| PR002581 | anti-ROR1 1015M3-H10(L:S93A) hIgG1 | 0.9741 | 6.73E-05 | 3182 | 42342 |
| PR002582 | anti-ROR1 1015M3-H10(H:D54E) hIgG1 | 0.798 | 1.93E-04 | 1991 | 32331 |
| PR002583 | anti-ROR1 1015M3-H10(H:D54E; L:N92Q) hIgG1 | 0.6725 | 2.23E-04 | 3950 | 25337 |
| PR002584 | anti-ROR1 1015M3-H10(H:D54E; L:S93A) hIgG1 | 0.6897 | 3.01E-04 | 3176 | 36635 |
| PR002585 | anti-ROR1 1015M3-H10(H:G55A) hIgG1 | 0.7713 | 3.89E-04 | 2614 | 32721 |
| PR002586 | anti-ROR1 1015M3-H10(H:G55A; L:N92Q) hIgG1 | 0.7543 | 2.66E-04 | 5486 | 41934 |
| PR002587 | anti-ROR1 1015M3-H10(H:G55A; L:S93A) hIgG1 | 0.7556 | 3.94E-04 | 2951 | 41531 |

### Example 5. Effect Data

### a. Results of ELISA assay (the ELISA assay was performed according to the same procedures as in Example 2c, except that the added samples were recombinant antibodies and the secondary antibody was a-Fc (Genscript, Cat#: A01854))

Table 15 shows the effect data from ELISA assays for the binding of PR001352-PR001371 and PR001415-PR001419 to hROR1 and hROR2. It can be seen from the table that all 25 antibodies showed good specificity for binding to ROR1 and had no cross-reactivity with ROR2. The corresponding values in the table are the measured median fluorescence intensity (MFI).

**Table 15**

| Ab ID | huROR1-his | huROR2-his | Ab ID | huROR1-his | huROR2-his |
|---|---|---|---|---|---|
| PR001352 | 2.4765 | 0.0335 | PR001368 | 2.507 | 0.0255 |
| PR001353 | 2.5415 | 0.031 | PR001369 | 2.476 | 0.031 |
| PR001354 | 2.445 | 0.027 | PR001370 | 2.5255 | 0.0335 |
| PR001355 | 2.543 | 0.0285 | PR001371 | 2.385 | 0.0235 |
| PR001356 | 2.379 | 0.0305 | PR001415 | 2.6105 | 0.03 |
| PR001357 | 2.4965 | 0.04 | PR001416 | 2.453 | 0.0265 |
| PR001358 | 2.47 | 0.049 | PR001417 | 2.382 | 0.027 |
| PR001359 | 2.393 | 0.038 | PR001418 | 2.4235 | 0.042 |
| PR001360 | 2.44 | 0.0315 | PR001419 | 2.4385 | 0.025 |
| PR001361 | 2.283 | 0.028 | PR000374 | 2.485 | 0.022 |
| PR001362 | 2.2215 | 0.032 | hIgG1 iso | 0.036 | 0.041 |
| PR001363 | 2.4485 | 0.0305 | blank | 0.024 | 0.023 |
| PR001364 | 2.3825 | 0.024 | | | |
| PR001365 | 2.3485 | 0.0285 | | | |
| PR001366 | 2.238 | 0.0305 | | | |
| PR001367 | 2.3775 | 0.0235 | | | |

### b. ADCC assay

ROR1 antibodies were assayed for activity of eliciting an ADCC effect against A549 cells endogenously expressing human ROR1 by using a CytoTox 96^{®} non-radioactive cytotoxicity assay kit (Promega, Cat#: G1780). Human PBMCs (Miaotong, since effective results may not be obtained with PBMC donors alone due to individual differences, typically at least two donors were selected in parallel for one experiment, which were, in this example, numbered as donor 1 and donor 2, respectively) were centrifuged at 300 g for 5 min and cultured in a medium (RPMI1640 + 10% FBS) overnight. Target cells and human PBMCs were centrifuged at 300 g for 5 min, and then resuspended in a medium (RPMI1640 + 2% FBS). The density of the target cells was adjusted to 2×10⁵ cells/mL, the cell density of PBMCs was adjusted to at least 6×10⁶ cells/mL, and each of the two types of cells was added to a 96-well plate at 50 µL/well (the effector-to-target ratio was at least 30:1). The test antibodies were diluted to different concentrations with a medium (RPMI1640 + 2% FBS) and then added to each well. The samples were incubated at 37 °C for at least 4 h, and then a 10× Triton-X 100 lysis buffer (RPMI1640 + 2% FBS + 10% Triton-X 100) was added to the target cell maximum LDH release control well and volume correction control well. The mixture was well mixed and incubated at 37 °C for 0.5 h. The 96-well plate was centrifuged at 300 g for 5 min, and 50 µL of the supernatant was removed. LDH chromogenic solution was then added to the plate at a concentration of 50 µL/well. After the mixture was left away from light at room temperature for 20 min, the plate was read on MD StakMax (OD490). PR000374 was used as a positive control, and human Iso IgG1 (CrownBio, Cat#: C0001-4) antibody was used as a negative control. For the calculation of the results, corrected readings were calculated first. The reading of the medium background control well was subtracted from the readings of the experimental wells, target cell spontaneous release LDH control well and effector cell spontaneous release LDH control well, and then the reading of the volume correction control well was subtracted from the reading of the target cell maximum LDH release control well. ADCC activity (%) = (corrected reading of experimental well - corrected reading of effector cell spontaneous release LDH control well - corrected reading of target cell spontaneous release LDH control well)/(corrected reading of target cell maximum LDH release control well - corrected reading of target cell spontaneous release LDH control well) × 100.

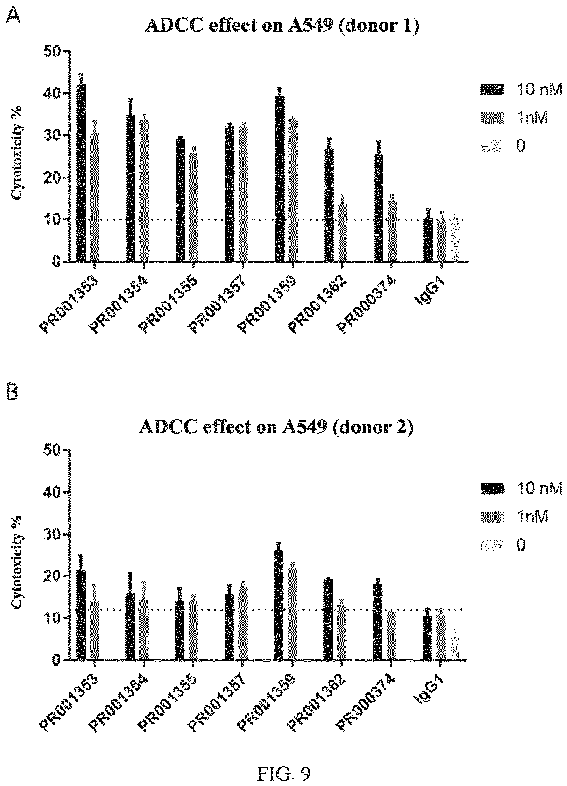

FIG. 9 and Table 16 show the ADCC effects of recombinant antibodies on an A549 cell line; wherein, A of FIG. 9 shows the results for PR001353, PR001354, PR001355, PR001357, PR001359 and PR001362 of ROR1-donor 1, and B of FIG. 9 shows the results for PR001353, PR001354, PR001355, PR001357, PR001359 and PR001362 of ROR1-donor 2. It can be seen from the figure and table that the antibodies PR001353, PR001354, PR001355, PR001357, PR001359 and PR001362 all had an ADCC effect superior to that of the reference antibody PR000374 in the prior art, wherein PR001359 showed the best ADCC effect, followed by PR001353 and PR001357.

**Table 16**

| | Cytotoxicity (%) | | | | | |
|---|---|---|---|---|---|---|
| | Donor 1 | | | Donor 2 | | |
| | 10 nM | 1 nM | 0 | 10 nM | 1 nM | 0 |
| PR001353 | 42.29 | 30.54 | | 21.49 | 14.05 | |
| PR001354 | 34.73 | 33.54 | | 15.92 | 14.36 | |
| PR001355 | 28.97 | 25.69 | | 14.20 | 14.14 | |
| PR001357 | 32.08 | 32.15 | | 15.81 | 17.45 | |
| PR001359 | 39.49 | 33.74 | | 26.15 | 21.93 | |
| PR001362 | 26.95 | 13.89 | | 19.25 | 13.23 | |
| PR000374 | 25.40 | 14.36 | | 18.19 | 11.44 | |
| IgG1 | 10.39 | 9.89 | 10.39 | 10.53 | 10.83 | 10.39 |

### c. CDC assay

ROR1 antibodies were assayed for ability to elicit a CDC effect against HEK293T-hROR1 cells by using a CellTiter-Glo luminescent cell viability assay kit (Promega, Cat#: G7573). The target cells HEK293T-hROR1 were centrifuged at 300 g for 5 min, and then resuspended in a DMEM serum-free medium. The density of the target cells was adjusted to 4e5 cells/mL, and the cell suspension was added to a 96-well plate at 25 µL/well. The antibodies were diluted to different concentrations with a serum-free medium, and the antibody dilution was added to a 96-well plate at 25 µL/well. 50 µL of normal human serum (GemCell, Cat#: 100-512) was added to give a final concentration of 50%, and the obtained mixture was incubated at 37 °C for 24 h. The 96-well plate was left to stand at room temperature for 30 min, and CellTiter-Glo chromogenic solution was added at 100 µL/well at room temperature. Then, the sample was incubated away from light at room temperature for 10 min. The plate was read with PE Enspire. CDC activity (%) = [1 - (luminescent sample)/(luminescent mock control)] × 100. PR000374 was used as a positive control, and human Iso IgG1 (CrownBio, Cat#: C0001-4) antibody was used as a negative control.

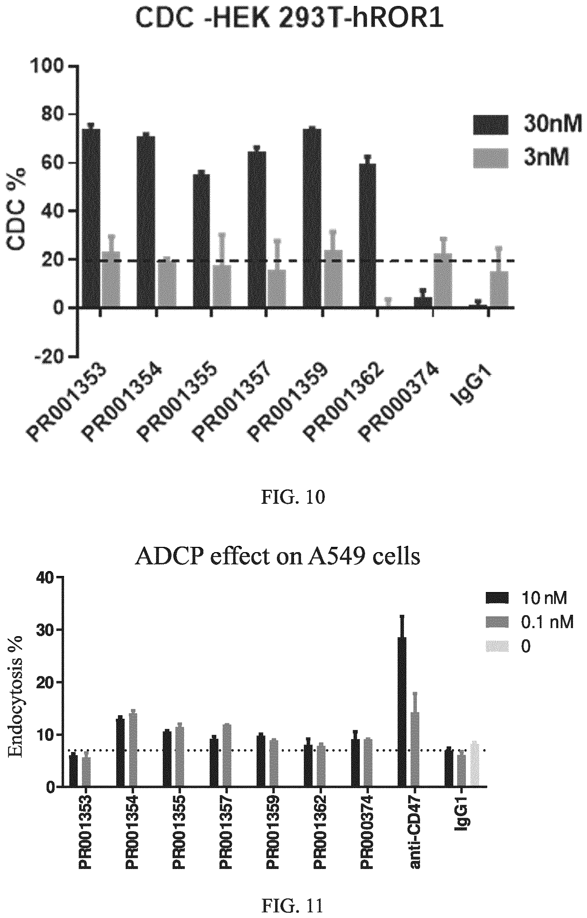

FIG. 10 illustrates the CDC effects of recombinant antibodies on HEK293T-hROR1. It can be seen from the figure that the antibodies PR001353, PR001354, PR001355, PR001357, PR001359 and PR001362 all showed good CDC effects at a high concentration.

### d. ADCP assay

CD14+ monocytes were isolated from PBMCs (Miaotong, Cat#: PB050F) using human CD14 sorting magnetic beads (Meltenyi, 130-050-201)) and resuspended at a density of 1×10⁶ cells/mL in an RPMI1640 medium containing 10% FBS, and 100 ng/mL GM-CSF (PeproTech, Cat#: 300-03-A) was added. Monocytes were seeded in a 6-well plate at 2×10⁶ cells/well and incubated in a carbon dioxide incubator at 37 °C for 8 days to differentiate into macrophages. The medium (containing 100 ng/mL GM-CSF) was changed every 3-4 days. After 8 days, the macrophages were digested with pancreatin, and the pancreatin reaction was terminated with RPMI1640 containing 10% FBS. The cells were collected, washed once with PBS and resuspended in PBS to give a density of 1×10⁶ cells/mL. A549 cells were also collected and resuspended in PBS to give a density of 1×10⁶ cells/mL. Macrophages were stained with 0.1 µM Far-red (in PBS) and A549 cells were stained with 0.5 µM CFSE (in PBS) at 4 °C for 5 min. The stained cells were centrifuged and washed once with >20 mL of RPMI1640 + 10% FBS medium. The washed cells were resuspended in 1% BSA-RPMI1640 medium, and the cell density was adjusted to 1.6×10⁶ cells/mL. The A549 cells (4×10⁴ cells/well) and the macrophages (4×10⁴ cells/well) were both added to a 96-well V-plate (Corning, Cat#: 3894) at 25 µL/well. The antibodies were diluted with 1% BSA-RPMI1640 to intermediate concentrations of 20 nM and 0.2 nM, and the diluted antibodies were added to the same 96-well V-plate containing A549 cells and macrophages at 50 µL/well. The mixture was well mixed, and incubated at 37 °C for 1 h. FITC+ A549 cells and Alexa647+ macrophages were identified by flow cytometry using NOVOcyte (Acea, US). Data were analyzed using FlowJo software (Tree Star, Ashland, OR), and the percentage of double-stained cells was used to determine ADCP-mediated cell killing. FIG. 11 illustrates the ADCP effects of recombinant antibodies on A549 cells.

It can be seen from FIG. 11 and Table 17 below that the ADCP effects of the antibodies PR001354, PR001355 and PR001357 were relatively small, and the ADCP effects of the other antibodies PR001353, PR001359 and PR001362 were comparable to that of the reference antibody PR000374 in the prior art.

**Table 17**

| | 10 nM | | 0.1 nM | | 0 | |
|---|---|---|---|---|---|---|
| PR001353 | 5.72 | 6.4 | 4.83 | 6.52 | / | / |
| PR001354 | 12.72 | 13.39 | 13.58 | 14.63 | / | / |
| PR001355 | 10.46 | 10.77 | 10.97 | 12.07 | / | / |
| PR001357 | 9.67 | 8.76 | 11.93 | 11.94 | / | // |
| PR001359 | 10.1 | 9.62 | 8.83 | 9.07 | / | / |
| PR001362 | 6.92 | 9.21 | 8.24 | 7.53 | / | / |
| PR000374 | 10.54 | 7.69 | 9.19 | 9.05 | / | / |
| anti-CD47 | 32.54 | 24.58 | 10.71 | 17.85 | / | / |
| IgG1 | 6.53 | 7.46 | 6.79 | 5.59 | 7.83 | 8.54 |

### Discussion

25 fully human antibodies with good binding affinity to hROR1 and cynoROR1, which have almost no cross-reactivity with ROR2, have been immunized and screened in the present invention.

When these 25 antibodies were bound to a binding tumor cell line, except for PR001362, the other 24 antibodies all showed binding effects (lower EC₅₀) almost 1 log higher than that of the reference antibody in the prior art. All antibodies showed KD values comparable to that of the reference antibody in the prior art. All 25 antibodies showed a significant endocytosis effect on the stably transfected cell strains. Still other antibodies, such as PR001359, had an ADCC effect in tumor cells but had no significant CDC/ADCP effects. Based on their application in the direction of ADCC, monoclonal antibodies may be developed in the future.

## Claims

1. An ROR1-targeted antibody or an antigen-binding fragment thereof, comprising a VL and a VH; wherein,
the VL comprises the following CDRs or mutations thereof: VL CDR1 with an amino acid sequence as set forth in SEQ ID NO: 69, VL CDR2 with an amino acid sequence as set forth in SEQ ID NO: 78, and VL CDR3 with an amino acid sequence as set forth in SEQ ID NO: 84; and
the VH comprises the following CDRs or mutations thereof: VH CDR1 with an amino acid sequence as set forth in SEQ ID NO: 12, VH CDR2 with an amino acid sequence as set forth in SEQ ID NO: 33, and VH CDR3 with an amino acid sequence as set forth in SEQ ID NO: 53;
wherein the mutation is an insertion, deletion or substitution of 3, 2 or 1 amino acids on the amino acid sequences of the CDRs.

2. The ROR1-targeted antibody or the antigen-binding fragment thereof according to claim 1, wherein the VLCDR1 has mutations of 4, 3, 2 or 1 amino acid substitutions of T5S, I6M/V, N7S and N8S on the amino acid sequence as set forth in SEQ ID NO: 69, with an amino acid sequence preferably as set forth in any one of SEQ ID NOs: 70-74; and/or
the VL CDR2 has a mutation of an amino acid substitution of S4N on the amino acid sequence as set forth in SEQ ID NO: 78, with an amino acid sequence as set forth in SEQ ID NO: 79; and/or
the VL CDR3 has mutations of 2 or 1 amino acid substitutions of N4K/Q and S5L/I/A on the amino acid sequence as set forth in SEQ ID NO: 84, with an amino acid sequence preferably as set forth in any one of SEQ ID NOs: 85-87, SEQ ID NO: 184 and SEQ ID NO: 185; and/or the VH CDR1 has mutations of 3, 2 or 1 amino acid substitutions of T3S, S6T/R/N and Y7F/S on the amino acid sequence as set forth in SEQ ID NO: 12, with an amino acid sequence preferably as set forth in any one of SEQ ID NOs: 13-18; and/or
the VH CDR2 has mutations of 4, 3, 2 or 1 amino acid substitutions of D3E, G4A, S5I/V/G and D6S/N on the amino acid sequence as set forth in SEQ ID NO: 33, with an amino acid sequence preferably as set forth in any one of SEQ ID NO: 32, SEQ ID NOs: 34-36 and SEQ ID NOs: 178-183; and/or
the VH CDR3 has mutations of 3, 2 or 1 amino substitutions of E1D, Q2L and L8I on the amino acid sequence as set forth in SEQ ID NO: 53, with an amino acid sequence preferably as set forth in any one of SEQ ID NOs: 52, 54 and 55.

3. The ROR1-targeted antibody or the antigen-binding fragment thereof according to claim 1 or 2, wherein the VL CDR1 has an amino acid sequence as set forth in SEQ ID NO: 69, the VL CDR2 has an amino acid sequence as set forth in SEQ ID NO: 78, and the VL CDR3 has an amino acid sequence as set forth in SEQ ID NO: 84; the VL CDR1 has an amino acid sequence as set forth in SEQ ID NO: 70, the VL CDR2 has an amino acid sequence as set forth in SEQ ID NO: 78, and the VL CDR3 has an amino acid sequence as set forth in SEQ ID NO: 85; the VL CDR1 has an amino acid sequence as set forth in SEQ ID NO: 71, the VL CDR2 has an amino acid sequence as set forth in SEQ ID NO: 78, and the VL CDR3 has an amino acid sequence as set forth in SEQ ID NO: 84; the VL CDR1 has an amino acid sequence as set forth in SEQ ID NO: 72, the VL CDR2 has an amino acid sequence as set forth in SEQ ID NO: 78, and the VL CDR3 has an amino acid sequence as set forth in SEQ ID NO: 85; the VL CDR1 has an amino acid sequence as set forth in SEQ ID NO: 69, the VL CDR2 has an amino acid sequence as set forth in SEQ ID NO: 78, and the VL CDR3 has an amino acid sequence as set forth in SEQ ID NO: 86; the VL CDR1 has an amino acid sequence as set forth in SEQ ID NO: 73, the VL CDR2 has an amino acid sequence as set forth in SEQ ID NO: 79, and the VL CDR3 has an amino acid sequence as set forth in SEQ ID NO: 85; the VL CDR1 has an amino acid sequence as set forth in SEQ ID NO: 74, the VL CDR2 has an amino acid sequence as set forth in SEQ ID NO: 78, and the VL CDR3 has an amino acid sequence as set forth in SEQ ID NO: 85; the VL CDR1 has an amino acid sequence as set forth in SEQ ID NO: 69, the VL CDR2 has an amino acid sequence as set forth in SEQ ID NO: 78, and the VL CDR3 has an amino acid sequence as set forth in SEQ ID NO: 87; the VL CDR1 has an amino acid sequence as set forth in SEQ ID NO: 73, the VL CDR2 has an amino acid sequence as set forth in SEQ ID NO: 78, and the VL CDR3 has an amino acid sequence as set forth in SEQ ID NO: 85; the VL CDR1 has an amino acid sequence as set forth in SEQ ID NO: 72, the VL CDR2 has an amino acid sequence as set forth in SEQ ID NO: 78, and the VL CDR3 has an amino acid sequence as set forth in SEQ ID NO: 84; the VL CDR1 has an amino acid sequence as set forth in SEQ ID NO: 73, the VL CDR2 has an amino acid sequence as set forth in SEQ ID NO: 78, and the VL CDR3 has an amino acid sequence as set forth in SEQ ID NO: 84; or the VL CDR1 has an amino acid sequence as set forth in SEQ ID NO: 70, the VL CDR2 has an amino acid sequence as set forth in SEQ ID NO: 78, and the VL CDR3 has an amino acid sequence as set forth in SEQ ID NO: 84; or the VL CDR1 has an amino acid sequence as set forth in SEQ ID NO: 69, the VL CDR2 has an amino acid sequence as set forth in SEQ ID NO: 78, and the VL CDR3 has an amino acid sequence as set forth in SEQ ID NO: 184; or the VL CDR1 has an amino acid sequence as set forth in SEQ ID NO: 69, the VL CDR2 has an amino acid sequence as set forth in SEQ ID NO: 78, and the VL CDR3 has an amino acid sequence as set forth in SEQ ID NO: 185; or the VL CDR1 has an amino acid sequence as set forth in SEQ ID NO: 72, the VL CDR2 has an amino acid sequence as set forth in SEQ ID NO: 78, and the VL CDR3 has an amino acid sequence as set forth in SEQ ID NO: 85; and/or
the VH CDR1 has an amino acid sequence as set forth in SEQ ID NO: 12, the VH CDR2 has an amino acid sequence as set forth in SEQ ID NO: 32, and the VH CDR3 has an amino acid sequence as set forth in SEQ ID NO: 52; the VH CDR1 has an amino acid sequence as set forth in SEQ ID NO: 12, the VH CDR2 has an amino acid sequence as set forth in SEQ ID NO: 32, and the VH CDR3 has an amino acid sequence as set forth in SEQ ID NO: 53; the VH CDR1 has an amino acid sequence as set forth in SEQ ID NO: 12, the VH CDR2 has an amino acid sequence as set forth in SEQ ID NO: 33, and the VH CDR3 has an amino acid sequence as set forth in SEQ ID NO: 53; the VH CDR1 has an amino acid sequence as set forth in SEQ ID NO: 12, the VH CDR2 has an amino acid sequence as set forth in SEQ ID NO: 34, and the VH CDR3 has an amino acid sequence as set forth in SEQ ID NO: 53; the VH CDR1 has an amino acid sequence as set forth in SEQ ID NO: 12, the VH CDR2 has an amino acid sequence as set forth in SEQ ID NO: 35, and the VH CDR3 has an amino acid sequence as set forth in SEQ ID NO: 53; the VH CDR1 has an amino acid sequence as set forth in SEQ ID NO: 13, the VH CDR2 has an amino acid sequence as set forth in SEQ ID NO: 33, and the VH CDR3 has an amino acid sequence as set forth in SEQ ID NO: 53; the VH CDR1 has an amino acid sequence as set forth in SEQ ID NO: 14, the VH CDR2 has an amino acid sequence as set forth in SEQ ID NO: 35, and the VH CDR3 has an amino acid sequence as set forth in SEQ ID NO: 53; the VH CDR1 has an amino acid sequence as set forth in SEQ ID NO: 13, the VH CDR2 has an amino acid sequence as set forth in SEQ ID NO: 33, and the VH CDR3 has an amino acid sequence as set forth in SEQ ID NO: 54; the VH CDR1 has an amino acid sequence as set forth in SEQ ID NO: 15, the VH CDR2 has an amino acid sequence as set forth in SEQ ID NO: 36, and the VH CDR3 has an amino acid sequence as set forth in SEQ ID NO: 53; the VH CDR1 has an amino acid sequence as set forth in SEQ ID NO: 16, the VH CDR2 has an amino acid sequence as set forth in SEQ ID NO: 33, and the VH CDR3 has an amino acid sequence as set forth in SEQ ID NO: 53; the VH CDR1 has an amino acid sequence as set forth in SEQ ID NO: 12, the VH CDR2 has an amino acid sequence as set forth in SEQ ID NO: 32, and the VH CDR3 has an amino acid sequence as set forth in SEQ ID NO: 55; the VH CDR1 has an amino acid sequence as set forth in SEQ ID NO: 17, the VH CDR2 has an amino acid sequence as set forth in SEQ ID NO: 34, and the VH CDR3 has an amino acid sequence as set forth in SEQ ID NO: 53; or the VH CDR1 has an amino acid sequence as set forth in SEQ ID NO: 18, the VH CDR2 has an amino acid sequence as set forth in SEQ ID NO: 34, and the VH CDR3 has an amino acid sequence as set forth in SEQ ID NO: 53; or the VH CDR1 has an amino acid sequence as set forth in SEQ ID NO: 12, the VH CDR2 has an amino acid sequence as set forth in SEQ ID NO: 178, and the VH CDR3 has an amino acid sequence as set forth in SEQ ID NO: 53; or the VH CDR1 has an amino acid sequence as set forth in SEQ ID NO: 12, the VH CDR2 has an amino acid sequence as set forth in SEQ ID NO: 179, and the VH CDR3 has an amino acid sequence as set forth in SEQ ID NO: 53; or the VH CDR1 has an amino acid sequence as set forth in SEQ ID NO: 12, the VH CDR2 has an amino acid sequence as set forth in SEQ ID NO: 180, and the VH CDR3 has an amino acid sequence as set forth in SEQ ID NO: 53; or the VH CDR1 has an amino acid sequence as set forth in SEQ ID NO: 12, the VH CDR2 has an amino acid sequence as set forth in SEQ ID NO: 181, and the VH CDR3 has an amino acid sequence as set forth in SEQ ID NO: 53; or the VH CDR1 has an amino acid sequence as set forth in SEQ ID NO: 16, the VH CDR2 has an amino acid sequence as set forth in SEQ ID NO: 178, and the VH CDR3 has an amino acid sequence as set forth in SEQ ID NO: 53; or the VH CDR1 has an amino acid sequence as set forth in SEQ ID NO: 16, the VH CDR2 has an amino acid sequence as set forth in SEQ ID NO: 179, and the VH CDR3 has an amino acid sequence as set forth in SEQ ID NO: 53; or the VH CDR1 has an amino acid sequence as set forth in SEQ ID NO: 17, the VH CDR2 has an amino acid sequence as set forth in SEQ ID NO: 182, and the VH CDR3 has an amino acid sequence as set forth in SEQ ID NO: 53; or
the VH CDR1 has an amino acid sequence as set forth in SEQ ID NO: 17, the VH CDR2 has an amino acid sequence as set forth in SEQ ID NO: 183, and the VH CDR3 has an amino acid sequence as set forth in SEQ ID NO: 53;
preferably, in the antibody or the antigen-binding fragment thereof,
the VL comprises VL CDR1, VL CDR2 and VL CDR3 with amino acid sequences as set forth in SEQ ID NOs: 69, 78 and 84, respectively; and the VH comprises VH CDR1, VH CDR2 and VH CDR3 with amino acid sequences as set forth in SEQ ID NOs: 12, 32 and 52, respectively;
or
the VL comprises VL CDR1, VL CDR2 and VL CDR3 with amino acid sequences as set forth in SEQ ID NOs: 70, 78 and 85, respectively; and the VH comprises VH CDR1, VH CDR2 and VH CDR3 with amino acid sequences as set forth in SEQ ID NOs: 12, 32 and 53, respectively;
or
the VL comprises VL CDR1, VL CDR2 and VL CDR3 with amino acid sequences as set forth in SEQ ID NOs: 71, 78 and 84, respectively; and the VH comprises VH CDR1, VH CDR2 and VH CDR3 with amino acid sequences as set forth in SEQ ID NOs: 12, 34 and 53, respectively;
or
the VL comprises VL CDR1, VL CDR2 and VL CDR3 with amino acid sequences as set forth in SEQ ID NOs: 72, 78 and 85, respectively; and the VH comprises VH CDR1, VH CDR2 and VH CDR3 with amino acid sequences as set forth in SEQ ID NOs: 12, 35 and 53, respectively;
or
the VL comprises VL CDR1, VL CDR2 and VL CDR3 with amino acid sequences as set forth in SEQ ID NOs: 69, 78 and 86, respectively; and the VH comprises VH CDR1, VH CDR2 and VH CDR3 with amino acid sequences as set forth in SEQ ID NOs: 12, 34 and 53, respectively;
or
the VL comprises VL CDR1, VL CDR2 and VL CDR3 with amino acid sequences as set forth in SEQ ID NOs: 73, 79 and 85, respectively; and the VH comprises VH CDR1, VH CDR2 and VH CDR3 with amino acid sequences as set forth in SEQ ID NOs: 12, 34 and 53, respectively;
or
the VL comprises VL CDR1, VL CDR2 and VL CDR3 with amino acid sequences as set forth in SEQ ID NOs: 74, 78 and 85, respectively; and the VH comprises VH CDR1, VH CDR2 and VH CDR3 with amino acid sequences as set forth in SEQ ID NOs: 13, 33 and 53, respectively;
or
the VL comprises VL CDR1, VL CDR2 and VL CDR3 with amino acid sequences as set forth in SEQ ID NOs: 69, 78 and 87, respectively; and the VH comprises VH CDR1, VH CDR2 and VH CDR3 with amino acid sequences as set forth in SEQ ID NOs: 12, 33 and 53, respectively;
or
the VL comprises VL CDR1, VL CDR2 and VL CDR3 with amino acid sequences as set forth in SEQ ID NOs: 73, 78 and 85, respectively; and the VH comprises VH CDR1, VH CDR2 and VH CDR3 with amino acid sequences as set forth in SEQ ID NOs: 14, 35 and 53, respectively;
or
the VL comprises VL CDR1, VL CDR2 and VL CDR3 with amino acid sequences as set forth in SEQ ID NOs: 73, 78 and 85, respectively; and the VH comprises VH CDR1, VH CDR2 and VH CDR3 with amino acid sequences as set forth in SEQ ID NOs: 13, 33 and 54, respectively;
or
the VL comprises VL CDR1, VL CDR2 and VL CDR3 with amino acid sequences as set forth in SEQ ID NOs: 69, 78 and 84, respectively; and the VH comprises VH CDR1, VH CDR2 and VH CDR3 with amino acid sequences as set forth in SEQ ID NOs: 15, 36 and 53, respectively;
or
the VL comprises VL CDR1, VL CDR2 and VL CDR3 with amino acid sequences as set forth in SEQ ID NOs: 69, 78 and 84, respectively; and the VH comprises VH CDR1, VH CDR2 and VH CDR3 with amino acid sequences as set forth in SEQ ID NOs: 16, 33 and 53, respectively;
or
the VL comprises VL CDR1, VL CDR2 and VL CDR3 with amino acid sequences as set forth in SEQ ID NOs: 69, 78 and 86, respectively; and the VH comprises VH CDR1, VH CDR2 and VH CDR3 with amino acid sequences as set forth in SEQ ID NOs: 12, 33 and 53, respectively;
or
the VL comprises VL CDR1, VL CDR2 and VL CDR3 with amino acid sequences as set forth in SEQ ID NOs: 73, 78 and 85, respectively; and the VH comprises VH CDR1, VH CDR2 and VH CDR3 with amino acid sequences as set forth in SEQ ID NOs: 12, 32 and 55, respectively;
or
the VL comprises VL CDR1, VL CDR2 and VL CDR3 with amino acid sequences as set forth in SEQ ID NOs: 73, 78 and 85, respectively; and the VH comprises VH CDR1, VH CDR2 and VH CDR3 with amino acid sequences as set forth in SEQ ID NOs: 13, 33 and 53, respectively;
or
the VL comprises VL CDR1, VL CDR2 and VL CDR3 with amino acid sequences as set forth in SEQ ID NOs: 69, 78 and 84, respectively; and the VH comprises VH CDR1, VH CDR2 and VH CDR3 with amino acid sequences as set forth in SEQ ID NOs: 17, 34 and 53, respectively;
or
the VL comprises VL CDR1, VL CDR2 and VL CDR3 with amino acid sequences as set forth in SEQ ID NOs: 72, 78 and 84, respectively; and the VH comprises VH CDR1, VH CDR2 and VH CDR3 with amino acid sequences as set forth in SEQ ID NOs: 12, 32 and 52, respectively;
or
the VL comprises VL CDR1, VL CDR2 and VL CDR3 with amino acid sequences as set forth in SEQ ID NOs: 69, 78 and 84, respectively; and the VH comprises VH CDR1, VH CDR2 and VH CDR3 with amino acid sequences as set forth in SEQ ID NOs: 12, 35 and 53, respectively;
or
the VL comprises VL CDR1, VL CDR2 and VL CDR3 with amino acid sequences as set forth in SEQ ID NOs: 69, 78 and 84, respectively; and the VH comprises VH CDR1, VH CDR2 and VH CDR3 with amino acid sequences as set forth in SEQ ID NOs: 18, 34 and 53, respectively;
or
the VL comprises VL CDR1, VL CDR2 and VL CDR3 with amino acid sequences as set forth in SEQ ID NOs: 69, 78 and 84, respectively; and the VH comprises VH CDR1, VH CDR2 and VH CDR3 with amino acid sequences as set forth in SEQ ID NOs: 13, 33 and 53, respectively;
or
the VL comprises VL CDR1, VL CDR2 and VL CDR3 with amino acid sequences as set forth in SEQ ID NOs: 73, 78 and 84, respectively; and the VH comprises VH CDR1, VH CDR2 and VH CDR3 with amino acid sequences as set forth in SEQ ID NOs: 15, 36 and 53, respectively;
or
the VL comprises VL CDR1, VL CDR2 and VL CDR3 with amino acid sequences as set forth in SEQ ID NOs: 70, 78 and 84, respectively; and the VH comprises VH CDR1, VH CDR2 and VH CDR3 with amino acid sequences as set forth in SEQ ID NOs: 12, 32 and 53, respectively;
or
the VL comprises VL CDR1, VL CDR2 and VL CDR3 with amino acid sequences as set forth in SEQ ID NOs: 69, 78 and 184, respectively; and the VH comprises VH CDR1, VH CDR2 and VH CDR3 with amino acid sequences as set forth in SEQ ID NOs: 12, 33 and 53, respectively;
or
the VL comprises VL CDR1, VL CDR2 and VL CDR3 with amino acid sequences as set forth in SEQ ID NOs: 69, 78 and 185, respectively; and the VH comprises VH CDR1, VH CDR2 and VH CDR3 with amino acid sequences as set forth in SEQ ID NOs: 12, 33 and 53, respectively;
or
the VL comprises VL CDR1, VL CDR2 and VL CDR3 with amino acid sequences as set forth in SEQ ID NOs: 69, 78 and 84, respectively; and the VH comprises VH CDR1, VH CDR2 and VH CDR3 with amino acid sequences as set forth in SEQ ID NOs: 12, 178 and 53, respectively;
or
the VL comprises VL CDR1, VL CDR2 and VL CDR3 with amino acid sequences as set forth in SEQ ID NOs: 69, 78 and 184, respectively; and the VH comprises VH CDR1, VH CDR2 and VH CDR3 with amino acid sequences as set forth in SEQ ID NOs: 12, 178 and 53, respectively;
or
the VL comprises VL CDR1, VL CDR2 and VL CDR3 with amino acid sequences as set forth in SEQ ID NOs: 69, 78 and 185, respectively; and the VH comprises VH CDR1, VH CDR2 and VH CDR3 with amino acid sequences as set forth in SEQ ID NOs: 12, 178 and 53, respectively;
or
the VL comprises VL CDR1, VL CDR2 and VL CDR3 with amino acid sequences as set forth in SEQ ID NOs: 69, 78 and 84, respectively; and the VH comprises VH CDR1, VH CDR2 and VH CDR3 with amino acid sequences as set forth in SEQ ID NOs: 12, 179 and 53, respectively;
or
the VL comprises VL CDR1, VL CDR2 and VL CDR3 with amino acid sequences as set forth in SEQ ID NOs: 69, 78 and 184, respectively; and the VH comprises VH CDR1, VH CDR2 and VH CDR3 with amino acid sequences as set forth in SEQ ID NOs: 12, 179 and 53, respectively;
or
the VL comprises VL CDR1, VL CDR2 and VL CDR3 with amino acid sequences as set forth in SEQ ID NOs: 69, 78 and 185, respectively; and the VH comprises VH CDR1, VH CDR2 and VH CDR3 with amino acid sequences as set forth in SEQ ID NOs: 12, 179 and 53, respectively;
or
the VL comprises VL CDR1, VL CDR2 and VL CDR3 with amino acid sequences as set forth in SEQ ID NOs: 72, 78 and 85, respectively; and the VH comprises VH CDR1, VH CDR2 and VH CDR3 with amino acid sequences as set forth in SEQ ID NOs: 12, 180 and 53, respectively;
or
the VL comprises VL CDR1, VL CDR2 and VL CDR3 with amino acid sequences as set forth in SEQ ID NOs: 72, 78 and 85, respectively; and the VH comprises VH CDR1, VH CDR2 and VH CDR3 with amino acid sequences as set forth in SEQ ID NOs: 12, 181 and 53, respectively;
or
the VL comprises VL CDR1, VL CDR2 and VL CDR3 with amino acid sequences as set forth in SEQ ID NOs: 69, 78 and 184, respectively; and the VH comprises VH CDR1, VH CDR2 and VH CDR3 with amino acid sequences as set forth in SEQ ID NOs: 16, 33 and 53, respectively;
or
the VL comprises VL CDR1, VL CDR2 and VL CDR3 with amino acid sequences as set forth in SEQ ID NOs: 69, 78 and 185, respectively; and the VH comprises VH CDR1, VH CDR2 and VH CDR3 with amino acid sequences as set forth in SEQ ID NOs: 16, 33 and 53, respectively;
or
the VL comprises VL CDR1, VL CDR2 and VL CDR3 with amino acid sequences as set forth in SEQ ID NOs: 69, 78 and 84, respectively; and the VH comprises VH CDR1, VH CDR2 and VH CDR3 with amino acid sequences as set forth in SEQ ID NOs: 16, 178 and 53, respectively;
or
the VL comprises VL CDR1, VL CDR2 and VL CDR3 with amino acid sequences as set forth in SEQ ID NOs: 69, 78 and 184, respectively; and the VH comprises VH CDR1, VH CDR2 and VH CDR3 with amino acid sequences as set forth in SEQ ID NOs: 16, 178 and 53, respectively;
or
the VL comprises VL CDR1, VL CDR2 and VL CDR3 with amino acid sequences as set forth in SEQ ID NOs: 69, 78 and 185, respectively; and the VH comprises VH CDR1, VH CDR2 and VH CDR3 with amino acid sequences as set forth in SEQ ID NOs: 16, 178 and 53, respectively;
or
the VL comprises VL CDR1, VL CDR2 and VL CDR3 with amino acid sequences as set forth in SEQ ID NOs: 69, 78 and 84, respectively; and the VH comprises VH CDR1, VH CDR2 and VH CDR3 with amino acid sequences as set forth in SEQ ID NOs: 16, 179 and 53, respectively;
or
the VL comprises VL CDR1, VL CDR2 and VL CDR3 with amino acid sequences as set forth in SEQ ID NOs: 69, 78 and 184, respectively; and the VH comprises VH CDR1, VH CDR2 and VH CDR3 with amino acid sequences as set forth in SEQ ID NOs: 16, 179 and 53, respectively;
or
the VL comprises VL CDR1, VL CDR2 and VL CDR3 with amino acid sequences as set forth in SEQ ID NOs: 69, 78 and 185, respectively; and the VH comprises VH CDR1, VH CDR2 and VH CDR3 with amino acid sequences as set forth in SEQ ID NOs: 16, 179 and 53, respectively;
or
the VL comprises VL CDR1, VL CDR2 and VL CDR3 with amino acid sequences as set forth in SEQ ID NOs: 69, 78 and 184, respectively; and the VH comprises VH CDR1, VH CDR2 and VH CDR3 with amino acid sequences as set forth in SEQ ID NOs: 17, 34 and 53, respectively;
or
the VL comprises VL CDR1, VL CDR2 and VL CDR3 with amino acid sequences as set forth in SEQ ID NOs: 69, 78 and 185, respectively; and the VH comprises VH CDR1, VH CDR2 and VH CDR3 with amino acid sequences as set forth in SEQ ID NOs: 17, 34 and 53, respectively;
or
the VL comprises VL CDR1, VL CDR2 and VL CDR3 with amino acid sequences as set forth in SEQ ID NOs: 69, 78 and 84, respectively; and the VH comprises VH CDR1, VH CDR2 and VH CDR3 with amino acid sequences as set forth in SEQ ID NOs: 17, 182 and 53, respectively;
or
the VL comprises VL CDR1, VL CDR2 and VL CDR3 with amino acid sequences as set forth in SEQ ID NOs: 69, 78 and 184, respectively; and the VH comprises VH CDR1, VH CDR2 and VH CDR3 with amino acid sequences as set forth in SEQ ID NOs: 17, 182 and 53, respectively;
or
the VL comprises VL CDR1, VL CDR2 and VL CDR3 with amino acid sequences as set forth in SEQ ID NOs: 69, 78 and 185, respectively; and the VH comprises VH CDR1, VH CDR2 and VH CDR3 with amino acid sequences as set forth in SEQ ID NOs: 17, 182 and 53, respectively;
or
the VL comprises VL CDR1, VL CDR2 and VL CDR3 with amino acid sequences as set forth in SEQ ID NOs: 69, 78 and 84, respectively; and the VH comprises VH CDR1, VH CDR2 and VH CDR3 with amino acid sequences as set forth in SEQ ID NOs: 17, 183 and 53, respectively;
or
the VL comprises VL CDR1, VL CDR2 and VL CDR3 with amino acid sequences as set forth in SEQ ID NOs: 69, 78 and 184, respectively; and the VH comprises VH CDR1, VH CDR2 and VH CDR3 with amino acid sequences as set forth in SEQ ID NOs: 17, 183 and 53, respectively;
or
the VL comprises VL CDR1, VL CDR2 and VL CDR3 with amino acid sequences as set forth in SEQ ID NOs: 69, 78 and 185, respectively; and the VH comprises VH CDR1, VH CDR2 and VH CDR3 with amino acid sequences as set forth in SEQ ID NOs: 17, 183 and 53, respectively.

4. The ROR1-targeted antibody or the antigen-binding fragment thereof according to any one of claims 1-3, wherein
the VL comprises an amino acid sequence as set forth in SEQ ID NO: 117; and the VH comprises an amino acid sequence as set forth in SEQ ID NO: 93; or
the VL comprises an amino acid sequence as set forth in SEQ ID NO: 118; and the VH comprises an amino acid sequence as set forth in SEQ ID NO: 94; or
the VL comprises an amino acid sequence as set forth in SEQ ID NO: 119; and the VH comprises an amino acid sequence as set forth in SEQ ID NO: 95; or
the VL comprises an amino acid sequence as set forth in SEQ ID NO: 120; and the VH comprises an amino acid sequence as set forth in SEQ ID NO: 96; or
the VL comprises an amino acid sequence as set forth in SEQ ID NO: 121; and the VH comprises an amino acid sequence as set forth in SEQ ID NO: 97; or
the VL comprises an amino acid sequence as set forth in SEQ ID NO: 122; and the VH comprises an amino acid sequence as set forth in SEQ ID NO: 98; or
the VL comprises an amino acid sequence as set forth in SEQ ID NO: 123; and the VH comprises an amino acid sequence as set forth in SEQ ID NO: 99; or
the VL comprises an amino acid sequence as set forth in SEQ ID NO: 124; and the VH comprises an amino acid sequence as set forth in SEQ ID NO: 100; or
the VL comprises an amino acid sequence as set forth in SEQ ID NO: 125; and the VH comprises an amino acid sequence as set forth in SEQ ID NO: 101; or
the VL comprises an amino acid sequence as set forth in SEQ ID NO: 126; and the VH comprises an amino acid sequence as set forth in SEQ ID NO: 102; or
the VL comprises an amino acid sequence as set forth in SEQ ID NO: 127; and the VH comprises an amino acid sequence as set forth in SEQ ID NO: 103; or
the VL comprises an amino acid sequence as set forth in SEQ ID NO: 128; and the VH comprises an amino acid sequence as set forth in SEQ ID NO: 104; or
the VL comprises an amino acid sequence as set forth in SEQ ID NO: 129; and the VH comprises an amino acid sequence as set forth in SEQ ID NO: 105; or
the VL comprises an amino acid sequence as set forth in SEQ ID NO: 130; and the VH comprises an amino acid sequence as set forth in SEQ ID NO: 106; or
the VL comprises an amino acid sequence as set forth in SEQ ID NO: 126; and the VH comprises an amino acid sequence as set forth in SEQ ID NO: 107; or
the VL comprises an amino acid sequence as set forth in SEQ ID NO: 128; and the VH comprises an amino acid sequence as set forth in SEQ ID NO: 108; or
the VL comprises an amino acid sequence as set forth in SEQ ID NO: 127; and the VH comprises an amino acid sequence as set forth in SEQ ID NO: 109; or
the VL comprises an amino acid sequence as set forth in SEQ ID NO: 129; and the VH comprises an amino acid sequence as set forth in SEQ ID NO: 110; or
the VL comprises an amino acid sequence as set forth in SEQ ID NO: 131; and the VH comprises an amino acid sequence as set forth in SEQ ID NO: 93; or
the VL comprises an amino acid sequence as set forth in SEQ ID NO: 117; and the VH comprises an amino acid sequence as set forth in SEQ ID NO: 111; or
the VL comprises an amino acid sequence as set forth in SEQ ID NO: 129; and the VH comprises an amino acid sequence as set forth in SEQ ID NO: 112; or
the VL comprises an amino acid sequence as set forth in SEQ ID NO: 129; and the VH comprises an amino acid sequence as set forth in SEQ ID NO: 113; or
the VL comprises an amino acid sequence as set forth in SEQ ID NO: 132; and the VH comprises an amino acid sequence as set forth in SEQ ID NO: 108; or
the VL comprises an amino acid sequence as set forth in SEQ ID NO: 133; and the VH comprises an amino acid sequence as set forth in SEQ ID NO: 114; or
the VL comprises an amino acid sequence as set forth in SEQ ID NO: 134; and the VH comprises an amino acid sequence as set forth in SEQ ID NO: 115; or
the VL comprises an amino acid sequence as set forth in SEQ ID NO: 194; and the VH comprises an amino acid sequence as set forth in SEQ ID NO: 95; or
the VL comprises an amino acid sequence as set forth in SEQ ID NO: 195; and the VH comprises an amino acid sequence as set forth in SEQ ID NO: 95; or
the VL comprises an amino acid sequence as set forth in SEQ ID NO: 119; and the VH comprises an amino acid sequence as set forth in SEQ ID NO: 186; or
the VL comprises an amino acid sequence as set forth in SEQ ID NO: 194; and the VH comprises an amino acid sequence as set forth in SEQ ID NO: 186; or
the VL comprises an amino acid sequence as set forth in SEQ ID NO: 195; and the VH comprises an amino acid sequence as set forth in SEQ ID NO: 186; or
the VL comprises an amino acid sequence as set forth in SEQ ID NO: 119; and the VH comprises an amino acid sequence as set forth in SEQ ID NO: 187; or
the VL comprises an amino acid sequence as set forth in SEQ ID NO: 194; and the VH comprises an amino acid sequence as set forth in SEQ ID NO: 187; or
the VL comprises an amino acid sequence as set forth in SEQ ID NO: 195; and the VH comprises an amino acid sequence as set forth in SEQ ID NO: 187; or
the VL comprises an amino acid sequence as set forth in SEQ ID NO: 121; and the VH comprises an amino acid sequence as set forth in SEQ ID NO: 188; or
the VL comprises an amino acid sequence as set forth in SEQ ID NO: 121; and the VH comprises an amino acid sequence as set forth in SEQ ID NO: 189; or
the VL comprises an amino acid sequence as set forth in SEQ ID NO: 196; and the VH comprises an amino acid sequence as set forth in SEQ ID NO: 105; or
the VL comprises an amino acid sequence as set forth in SEQ ID NO: 197; and the VH comprises an amino acid sequence as set forth in SEQ ID NO: 105; or
the VL comprises an amino acid sequence as set forth in SEQ ID NO: 129; and the VH comprises an amino acid sequence as set forth in SEQ ID NO: 190; or
the VL comprises an amino acid sequence as set forth in SEQ ID NO: 196; and the VH comprises an amino acid sequence as set forth in SEQ ID NO: 190; or
the VL comprises an amino acid sequence as set forth in SEQ ID NO: 197; and the VH comprises an amino acid sequence as set forth in SEQ ID NO: 190; or
the VL comprises an amino acid sequence as set forth in SEQ ID NO: 129; and the VH comprises an amino acid sequence as set forth in SEQ ID NO: 191; or
the VL comprises an amino acid sequence as set forth in SEQ ID NO: 196; and the VH comprises an amino acid sequence as set forth in SEQ ID NO: 191; or
the VL comprises an amino acid sequence as set forth in SEQ ID NO: 197; and the VH comprises an amino acid sequence as set forth in SEQ ID NO: 191; or
the VL comprises an amino acid sequence as set forth in SEQ ID NO: 196; and the VH comprises an amino acid sequence as set forth in SEQ ID NO: 110; or
the VL comprises an amino acid sequence as set forth in SEQ ID NO: 197; and the VH comprises an amino acid sequence as set forth in SEQ ID NO: 110; or
the VL comprises an amino acid sequence as set forth in SEQ ID NO: 129; and the VH comprises an amino acid sequence as set forth in SEQ ID NO: 192; or
the VL comprises an amino acid sequence as set forth in SEQ ID NO: 196; and the VH comprises an amino acid sequence as set forth in SEQ ID NO: 192; or
the VL comprises an amino acid sequence as set forth in SEQ ID NO: 197; and the VH comprises an amino acid sequence as set forth in SEQ ID NO: 192; or
the VL comprises an amino acid sequence as set forth in SEQ ID NO: 129; and the VH comprises an amino acid sequence as set forth in SEQ ID NO: 193; or
the VL comprises an amino acid sequence as set forth in SEQ ID NO: 196; and the VH comprises an amino acid sequence as set forth in SEQ ID NO: 193; or
the VL comprises an amino acid sequence as set forth in SEQ ID NO: 197; and the VH comprises an amino acid sequence as set forth in SEQ ID NO: 193.

5. The ROR1-targeted antibody or the antigen-binding fragment thereof according to any one of claims 1-4, further comprising a heavy chain constant region and/or a light chain constant region; wherein
preferably, the heavy chain constant region is selected from hIgG1, hIgG2, hIgG3 or hIgG4, and the light chain constant region is selected from a κ chain or a λ chain; and
more preferably, the heavy chain constant region is hIgG1 and the light chain constant region is a κ chain of a human antibody.

6. The ROR1-targeted antibody or the antigen-binding fragment thereof according to any one of claims 1 to 5, being a full-length antibody; a Fab; a Fab'; an F(ab')₂; an Fv, preferably an scFv; a bispecific antibody; a multispecific antibody; a heavy-chain antibody or a single-domain antibody; or a monoclonal antibody or a polyclonal antibody derived from the above antibodies.

7. The ROR1-targeted antibody or the antigen-binding fragment thereof according to any one of claims 1-6, being a full-length antibody comprising a heavy chain and a light chain; wherein,
the heavy chain comprises an amino acid sequence as set forth in SEQ ID NO: 136; and the light chain comprises an amino acid sequence as set forth in SEQ ID NO: 160; or
the heavy chain comprises an amino acid sequence as set forth in SEQ ID NO: 137; and the light chain comprises an amino acid sequence as set forth in SEQ ID NO: 161; or
the heavy chain comprises an amino acid sequence as set forth in SEQ ID NO: 138; and the light chain comprises an amino acid sequence as set forth in SEQ ID NO: 162; or
the heavy chain comprises an amino acid sequence as set forth in SEQ ID NO: 139; and the light chain comprises an amino acid sequence as set forth in SEQ ID NO: 163; or
the heavy chain comprises an amino acid sequence as set forth in SEQ ID NO: 140; and the light chain comprises an amino acid sequence as set forth in SEQ ID NO: 164; or
the heavy chain comprises an amino acid sequence as set forth in SEQ ID NO: 141; and the light chain comprises an amino acid sequence as set forth in SEQ ID NO: 165; or
the heavy chain comprises an amino acid sequence as set forth in SEQ ID NO: 142; and the light chain comprises an amino acid sequence as set forth in SEQ ID NO: 166; or
the heavy chain comprises an amino acid sequence as set forth in SEQ ID NO: 143; and the light chain comprises an amino acid sequence as set forth in SEQ ID NO: 167; or
the heavy chain comprises an amino acid sequence as set forth in SEQ ID NO: 144; and the light chain comprises an amino acid sequence as set forth in SEQ ID NO: 168; or
the heavy chain comprises an amino acid sequence as set forth in SEQ ID NO: 145; and the light chain comprises an amino acid sequence as set forth in SEQ ID NO: 169; or
the heavy chain comprises an amino acid sequence as set forth in SEQ ID NO: 146; and the light chain comprises an amino acid sequence as set forth in SEQ ID NO: 170; or
the heavy chain comprises an amino acid sequence as set forth in SEQ ID NO: 147; and the light chain comprises an amino acid sequence as set forth in SEQ ID NO: 171; or
the heavy chain comprises an amino acid sequence as set forth in SEQ ID NO: 148; and the light chain comprises an amino acid sequence as set forth in SEQ ID NO: 172; or
the heavy chain comprises an amino acid sequence as set forth in SEQ ID NO: 149; and the light chain comprises an amino acid sequence as set forth in SEQ ID NO: 173; or
the heavy chain comprises an amino acid sequence as set forth in SEQ ID NO: 150; and the light chain comprises an amino acid sequence as set forth in SEQ ID NO: 169; or
the heavy chain comprises an amino acid sequence as set forth in SEQ ID NO: 151; and the light chain comprises an amino acid sequence as set forth in SEQ ID NO: 171; or
the heavy chain comprises an amino acid sequence as set forth in SEQ ID NO: 152; and the light chain comprises an amino acid sequence as set forth in SEQ ID NO: 170; or
the heavy chain comprises an amino acid sequence as set forth in SEQ ID NO: 153; and the light chain comprises an amino acid sequence as set forth in SEQ ID NO: 172; or
the heavy chain comprises an amino acid sequence as set forth in SEQ ID NO: 136; and the light chain comprises an amino acid sequence as set forth in SEQ ID NO: 174; or
the heavy chain comprises an amino acid sequence as set forth in SEQ ID NO: 154; and the light chain comprises an amino acid sequence as set forth in SEQ ID NO: 160; or
the heavy chain comprises an amino acid sequence as set forth in SEQ ID NO: 155; and the light chain comprises an amino acid sequence as set forth in SEQ ID NO: 172; or
the heavy chain comprises an amino acid sequence as set forth in SEQ ID NO: 156; and the light chain comprises an amino acid sequence as set forth in SEQ ID NO: 172; or
the heavy chain comprises an amino acid sequence as set forth in SEQ ID NO: 151; and the light chain comprises an amino acid sequence as set forth in SEQ ID NO: 175; or
the heavy chain comprises an amino acid sequence as set forth in SEQ ID NO: 157; and the light chain comprises an amino acid sequence as set forth in SEQ ID NO: 176; or
the heavy chain comprises an amino acid sequence as set forth in SEQ ID NO: 158; and the light chain comprises an amino acid sequence as set forth in SEQ ID NO: 177; or
the heavy chain comprises an amino acid sequence as set forth in SEQ ID NO: 138; and the light chain comprises an amino acid sequence as set forth in SEQ ID NO: 206; or
the heavy chain comprises an amino acid sequence as set forth in SEQ ID NO: 138; and the light chain comprises an amino acid sequence as set forth in SEQ ID NO: 207; or
the heavy chain comprises an amino acid sequence as set forth in SEQ ID NO: 198; and the light chain comprises an amino acid sequence as set forth in SEQ ID NO: 162; or
the heavy chain comprises an amino acid sequence as set forth in SEQ ID NO: 198; and the light chain comprises an amino acid sequence as set forth in SEQ ID NO: 206; or
the heavy chain comprises an amino acid sequence as set forth in SEQ ID NO: 198; and the light chain comprises an amino acid sequence as set forth in SEQ ID NO: 207; or
the heavy chain comprises an amino acid sequence as set forth in SEQ ID NO: 199; and the light chain comprises an amino acid sequence as set forth in SEQ ID NO: 162; or
the heavy chain comprises an amino acid sequence as set forth in SEQ ID NO: 199; and the light chain comprises an amino acid sequence as set forth in SEQ ID NO: 206; or
the heavy chain comprises an amino acid sequence as set forth in SEQ ID NO: 199; and the light chain comprises an amino acid sequence as set forth in SEQ ID NO: 207; or
the heavy chain comprises an amino acid sequence as set forth in SEQ ID NO: 200; and the light chain comprises an amino acid sequence as set forth in SEQ ID NO: 164; or
the heavy chain comprises an amino acid sequence as set forth in SEQ ID NO: 201; and the light chain comprises an amino acid sequence as set forth in SEQ ID NO: 164; or
the heavy chain comprises an amino acid sequence as set forth in SEQ ID NO: 148; and the light chain comprises an amino acid sequence as set forth in SEQ ID NO: 208; or
the heavy chain comprises an amino acid sequence as set forth in SEQ ID NO: 148; and the light chain comprises an amino acid sequence as set forth in SEQ ID NO: 209; or
the heavy chain comprises an amino acid sequence as set forth in SEQ ID NO: 202; and the light chain comprises an amino acid sequence as set forth in SEQ ID NO: 172; or
the heavy chain comprises an amino acid sequence as set forth in SEQ ID NO: 202; and the light chain comprises an amino acid sequence as set forth in SEQ ID NO: 208; or
the heavy chain comprises an amino acid sequence as set forth in SEQ ID NO: 202; and the light chain comprises an amino acid sequence as set forth in SEQ ID NO: 209; or
the heavy chain comprises an amino acid sequence as set forth in SEQ ID NO: 203; and the light chain comprises an amino acid sequence as set forth in SEQ ID NO: 172; or
the heavy chain comprises an amino acid sequence as set forth in SEQ ID NO: 203; and the light chain comprises an amino acid sequence as set forth in SEQ ID NO: 208; or
the heavy chain comprises an amino acid sequence as set forth in SEQ ID NO: 203; and the light chain comprises an amino acid sequence as set forth in SEQ ID NO: 209; or
the heavy chain comprises an amino acid sequence as set forth in SEQ ID NO: 153; and the light chain comprises an amino acid sequence as set forth in SEQ ID NO: 208; or
the heavy chain comprises an amino acid sequence as set forth in SEQ ID NO: 153; and the light chain comprises an amino acid sequence as set forth in SEQ ID NO: 209; or
the heavy chain comprises an amino acid sequence as set forth in SEQ ID NO: 204; and the light chain comprises an amino acid sequence as set forth in SEQ ID NO: 172; or
the heavy chain comprises an amino acid sequence as set forth in SEQ ID NO: 204; and the light chain comprises an amino acid sequence as set forth in SEQ ID NO: 208; or
the heavy chain comprises an amino acid sequence as set forth in SEQ ID NO: 204; and the light chain comprises an amino acid sequence as set forth in SEQ ID NO: 209; or
the heavy chain comprises an amino acid sequence as set forth in SEQ ID NO: 205; and the light chain comprises an amino acid sequence as set forth in SEQ ID NO: 172; or
the heavy chain comprises an amino acid sequence as set forth in SEQ ID NO: 205; and the light chain comprises an amino acid sequence as set forth in SEQ ID NO: 208; or
the heavy chain comprises an amino acid sequence as set forth in SEQ ID NO: 205; and the light chain comprises an amino acid sequence as set forth in SEQ ID NO: 209.

8. An isolated nucleic acid encoding the ROR1-targeted antibody or the antigen-binding fragment thereof according to any one of claims 1-7.

9. A recombinant expression vector comprising the isolated nucleic acid according to claim 8, preferably, the expression vector comprises a eukaryotic cell expression vector and/or a prokaryotic cell expression vector.

10. A transformant comprising the recombinant expression vector according to claim 9, preferably, the transformant has a host cell being a prokaryotic cell and/or a eukaryotic cell, wherein the prokaryotic cell is preferably an *E. coli* TG1 or BL21 cell, and the eukaryotic cell is preferably an HEK293 cell or a CHO cell.

11. A chimeric antigen receptor comprising the ROR1-targeted antibody or the antigen-binding fragment thereof according to any one of claims 1-7.

12. A genetically modified cell comprising the chimeric antigen receptor according to claim 11, preferably, the genetically modified cell is a eukaryotic cell, preferably an isolated human cell, and more preferably an immune cell such as a T cell or an NK cell.

13. A method for preparing the ROR1-targeted antibody or the antigen-binding fragment thereof, comprising the following steps: culturing the transformant according to claim 10, and obtaining the ROR1-targeted antibody or the antigen-binding fragment thereof from a culture.

14. An antibody-drug conjugate comprising a cytotoxic agent, and the ROR1-targeted antibody or the antigen-binding fragment thereof according to any one of claims 1-7.

15. A pharmaceutical composition comprising the ROR1-targeted antibody or the antigen-binding fragment thereof according to any one of claims 1-7, and/or the antibody-drug conjugate according to claim 14.

16. Use of the ROR1-targeted antibody or the antigen-binding fragment thereof according to any one of claims 1-7, the chimeric antigen receptor according to claim 11, the genetically modified cell according to claim 12, the antibody-drug conjugate according to claim 14, and/or the pharmaceutical composition according to claim 15 in the preparation of a medicament for the prevention and/or treatment of a cancer, wherein
preferably, the cancer is colon cancer, lung cancer, prostate cancer, liver cancer, kidney cancer, pancreatic cancer, breast cancer, cervical cancer, ovarian cancer, thyroid tumor, chronic lymphocytic leukemia, acute lymphocytic leukemia, acute myeloid leukemia, non-Hodgkin's lymphoma, chronic myeloid lymphocytic leukemia, multiple myeloma, melanoma, colorectal cancer, glioblastoma and/or endometrial cancer.

17. A kit comprising the antibody or the antigen-binding fragment thereof according to any one of claims 1-7, the chimeric antigen receptor according to claim 11, the genetically modified cell according to claim 12, or the antibody-drug conjugate according to claim 14 or the pharmaceutical composition according to claim 15,
preferably, the kit further comprises (i) a device for administering the antibody or the antigen-binding fragment thereof, the antibody-drug conjugate or the pharmaceutical composition; and/or (ii) instructions.

18. A method for detecting ROR1 in a sample, comprising conducting a detection using the ROR1-targeted antibody or the antigen-binding fragment thereof according to any one of claims 1-7, wherein the detection is preferably a diagnosis.

19. Use of the ROR1-targeted antibody or the antigen-binding fragment thereof according to any one of claims 1-7, the chimeric antigen receptor according to claim 11, the genetically modified cell according to claim 12, the antibody-drug conjugate according to claim 14, and/or the pharmaceutical composition according to claim 15 in the diagnosis, prevention and/or treatment of a cancer, wherein the cancer is preferably a cancer in the use according to claim 16.

20. A kit of parts comprising a kit A and a kit B, wherein the kit A comprises the ROR1-targeted antibody or the antigen-binding fragment thereof according to any one of claims 1-7, the chimeric antigen receptor according to claim 11, the genetically modified cell according to claim 12, the antibody-drug conjugate according to claim 14, and/or the pharmaceutical composition according to claim 15; the kit B is an additional drug, preferably a macromolecular drug such as an anti-cancer (anti-tumor) antibody or a pharmaceutical composition comprising the additional anti-cancer (anti-tumor) antibody, or a micromolecular drug; and
preferably, the kit A and the kit B can be used simultaneously, or the kit A can be used prior to the use of the kit B, or the kit B can be used prior to the use of the kit A.

21. A method for treating a cancer in a subject in need, comprising administering to the subject in need the ROR1-targeted antibody or the antigen-binding fragment thereof according to any one of claims 1-7, the chimeric antigen receptor according to claim 11, the genetically modified cell according to claim 12, the antibody-drug conjugate according to claim 14, and/or the pharmaceutical composition according to claim 15, wherein the cancer is preferably a cancer in the use according to claim 16; and
preferably, further comprising administering an additional drug, preferably a macromolecular drug such as an additional anti-cancer (anti-tumor) antibody or a pharmaceutical composition comprising the additional anti-cancer (anti-tumor) antibody, or a micromolecular drug.
